# EUROPEAN PATENT SPECIFICATION

(11) **EP 0 462 935 B1**
(45) Date of publication and mention of the grant of the patent: **19.07.1995**
(21) Application number: 91810477.9
(22) Date of filing: 19.06.1991
(51) Int. Cl.: C07F 9/655, A61K 31/685, C07D 307/12

(54) **Enantiomers of 2-tetrahydrofuran derivatives, intermediates therefor and their preparation**
2-Tetrahydrofuran-Enantiomerderivate, Zwischenprodukte davon und ihre Herstellung
Dérivés énantiomériques de 2-tétrahydrofuranne, leurs intermédiaires et leur préparation

(30) Priority: 19.06.1990 US 540438; 29.01.1991 US 647396
(43) Date of publication of application: 27.12.1991
(73) Proprietor: SANDOZ AG, 4002 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Estermann, Heinrich, CH-4123 Allschwil (CH); Houlihan, William Joseph, Mountain Lakes, N.J. 07046 (US); Kapa, Prasad Koteswara, Parsippany, N.J. 07054 (US); Underwood, Russell Lee, Randolph, N.J. 07869 (US)

(56) References cited:
- EP-A- 0 178 261
- EP-A- 0 213 082
- US-A- 4 868 319
- LIPIDS, vol. 22, no. 11, November 1987, pages 884-890, AN INTERNATIONAL CONFERENCE ON DIET, LIPIDS AND CANCER METABOLISM-MEMBRANES-MECHANISMS, YularaResort, Northern Territory, 22nd - 26th May 1988; W.J. HOULIHAN et al.:"Antitumor activity of SRI 62-834, a cyclic ether analog of ET-18-OCH3"

## Description

The invention relates to enantiomers of 2-tetrahydrofuran derivatives, their preparation, and intermediates therefor and their preparation.

It concerns in one aspect the **compound of formula I** 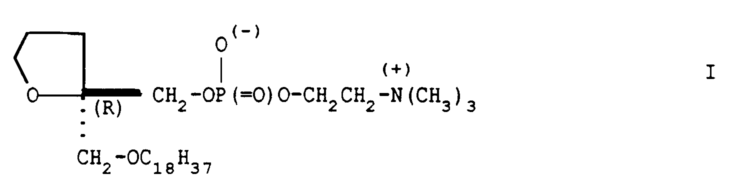
namely (R)-2-[(2-octadecyloxymethyltetrahydrofuran-2-ylmethoxy)hydroxyphosphinyloxy]-N,N,N-trimethylethanaminium hydroxide inner salt-4-oxide
or its **enantiomer** with the (S) configuration.

The racemate of the compound of formula I is known. It is disclosed e.g. in USP 4'673'672 as Example 5 on columns 23-24, and in its European counterpart EP-A-213082; the compounds in the USP are disclosed as being useful as anti-tumor agents.

It has now been found that the specific (R) optical isomer of formula I and its enantiomer, which had nowhere been specifically disclosed prior to the priority date of the present invention, have surprising and beneficial pharmacological properties, and that they can be obtained starting from (R)-2,2-bis(hydroxymethyl)tetrahydrofuran monobutyrate of formula II 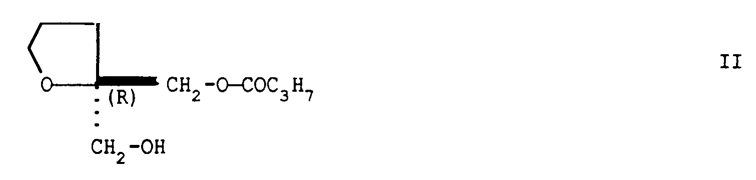
in protected form.

Thus in a further aspect the invention also concerns a **process** for the preparation of the compound of formula I and its enantiomer comprising octadecylating and phosphorylcholinating the compound of formula II, with intermediate protection and deprotection steps as appropriate.

Examples of the compound of formula II in protected form are compounds in which the hydroxy group is protected by a hydrolytically cleavable group, such as the compound of formula IIa 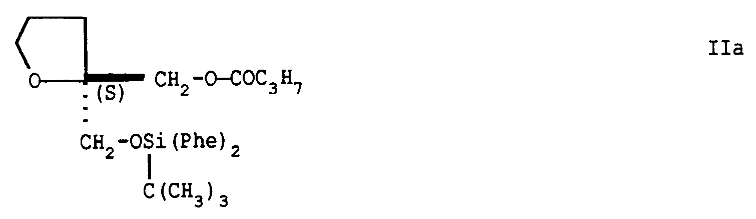
and the compound of formula IIb 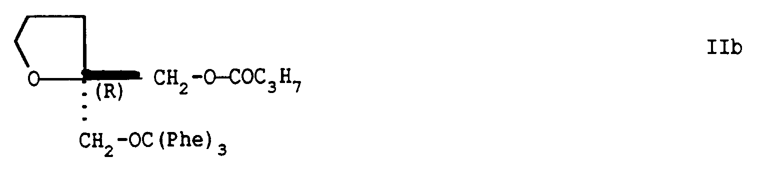
The compound of formula II in free or protected form is itself novel. In a further aspect the invention concerns the **compound of formula II in free form or in protected form**, and its preparation by a **process** comprising enantiospecifically monohydrolysing 2,2-bis(hydroxymethyl)tetrahydrofuran dibutyrate of formula III 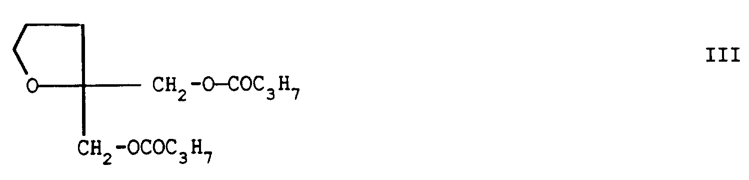
and optionally protecting the resultant compound of formula II in free form.

In a further aspect the invention thus also concerns the **use** of the compound of formula II **as an intermediate**, e.g. as an intermediate in preparing the compound of formula I or the enantiomer thereof.

Many pharmacologically active compounds occur as a mixture of optical isomers. Despite the fact that the desired pharmacological activity usually resides in one isomer, mixtures of optical isomers are employed because the prohibitive cost of separation of the isomers exceeds the potential advantage of a possible increase in activity. However, it is quite apparent that many pharmacologists are becoming increasingly aware of other implications in the administration of mixtures of optical isomers wherein one or more isomers have to be regarded as impurities which may not only be devoid of the desired therapeutic effect but may contribute unwanted physiological effects including toxicity.

Numerous research endeavors have been directed to processes for producing specific optical isomers of pharmacologically active compounds, and such processes are well documented. Prominent among these processes is the use of enzymes to induce stereospecific transformations. For example, in Chemistry Letters (1988) 1717-1720, there is described the enzyme-catalyzed asymmetrization of cis-2,5-bis(hydroxymethyl)tetrahydrofurans. More particularly, this reference describes the asymmetric hydrolysis of various diesters of cis-2,5-bis(hydroxymethyl)tetrahydrofuran catalyzed by pig liver esterase, porcine pancreas lipase or Candida cylindracea lipase. As can be seen from the results obtained, the yields are rather low with the exception of one particular case, Entry 1 in Table 1, where an 86 % yield was obtained.

In connection with the present invention it was desired to obtain optically pure intermediates derived from 2,2-bis(hydroxymethyl)tetrahydrofuran. As enzymatic processes are, in general, substrate-specific, there was a need to develop suitable methodology for the asymmetrization of 2,2-disubstituted tetrahydrofuran derivatives. In contrast to the 2,5-system, the optically pure monoacetates derived from the 2,2-system are prone to racemization via intramolecular acyl transfer. To this end, the process part of the present invention leading to the compound of formula II and its enantiomer represents a simple and economic process for preparing a specific optical isomer in good yields utilizing relatively small amounts of enzyme and mild reaction conditions. Thus the degree of optical purity attained is above 96 %, e.g. about 99 %. Chemical purity is equal to or above 99 %.

The isomer of formula II can be used for the preparation of further compounds in optically active form in addition to the compound of formula I and its enantiomer; the compound of formula II in free form and its protected forms are thus valuable intermediates in the preparation of specific pharmacologically active compounds in optically active form.

The **process of the invention** for the preparation of the **compound of formula I and its enantiomer** is effected using conventional techniques and reaction steps.

Thus the **compound of formula I** can be obtained e.g. according to the following reaction sequence: 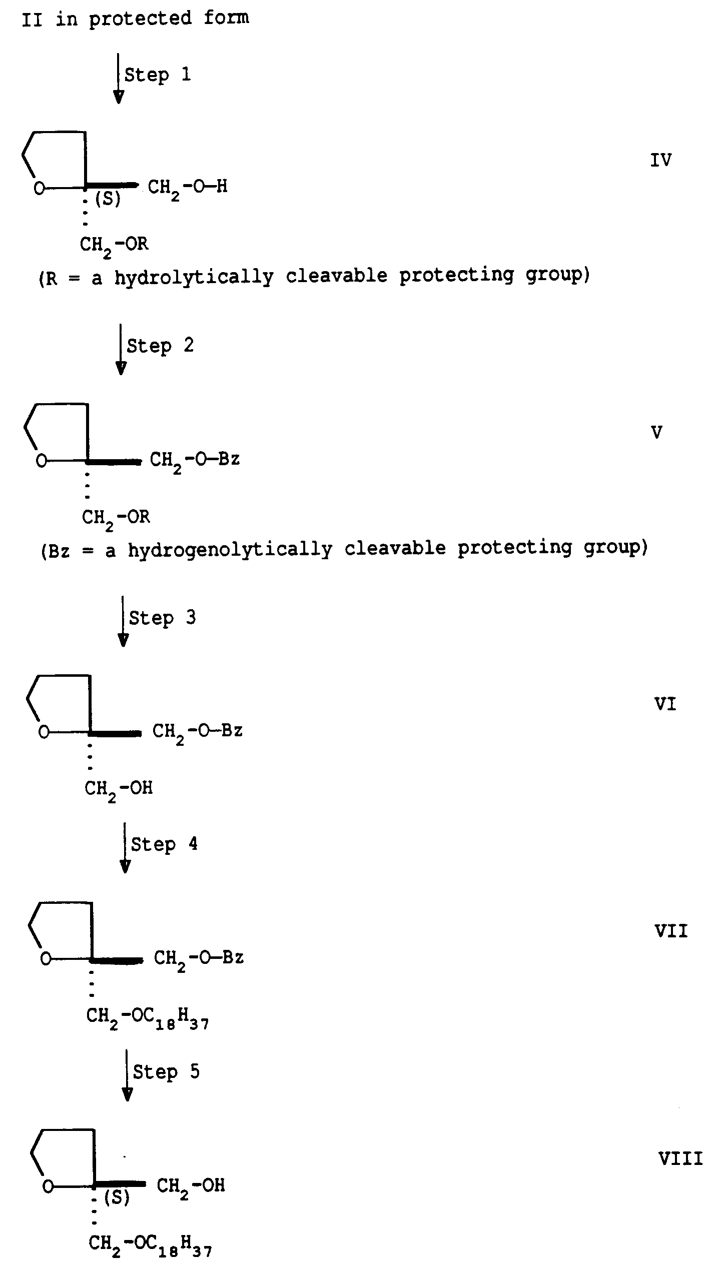 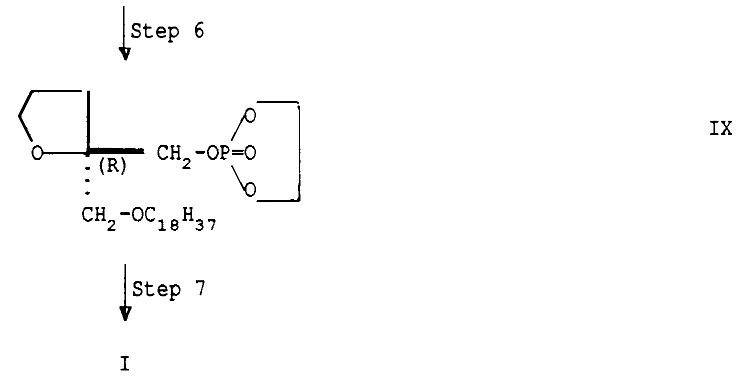
**Step 1** preferably is effected by alcoholic hydrolysis with e.g. methanol, in the presence of an organic base such as triethylamine or pyridine, or an inorganic base such as sodium or potassium carbonate. The reaction is carried out at a temperature of from about 0° to about 30°C. Suitable as protecting groups are especially hydrolytically cleavable groups such as silyl groups, e.g. tert-butyldiphenylsilyl, or bulky aralkyl groups such as triphenylmethyl (trityl). Preferred are the compounds of formula IIa and IIb.

**Step 2** concerns the reaction of the compound of formula IV with an appropriate reactive derivative such as benzyl bromide for the introduction of benzyl as hydrogenolytically cleavable protecting group Bz, preferably in the presence of sodium hydride. The reaction is conveniently carried out in an inert, organic solvent, e.g. a cyclic ether such as tetrahydrofuran, at a temperature of between about 0° and about 60°C or reflux temperature of the solvent. When the protecting group R is trityl the sodium hydride (e.g. a 60 % dispersion) is advantageously supplemented with tetrabutylammonium iodide. With benzyl as group Bz and tert-butyldiphenylsilyl as group R the compound of formula V is in the (S) configuration.

**Step 3** involves reaction with, preferably, tetrabutylammonium fluoride. This reaction is conveniently carried out in the presence of an inert, organic solvent, e.g. a cyclic ether such as tetrahydrofuran, or a lower alkyl nitrile such as acetonitrile. The temperature is from about 0° to about 30°C. With benzyl as group Bz the compound of formula VI has the (R) configuration. Alternatively the reaction may be effected with an aqueous solution of trifluoroacetic acid, conveniently in the presence of an inert, organic solvent, e.g. a halogenated hydrocarbon such as methylene chloride. The temperature is preferably between about 0° and about 40°C.

**Step 4** involves alkylation with an appropriate octadecane derivative such as 1-halooctadecane, e.g. 1-bromooctadecane, or the corresponding C₁₈-para-toluenesulfonate, in the presence of a base such as sodium hydroxide. The reaction is conveniently carried out in the presence of an inert organic solvent, e.g. a dialkylamide such as dimethylformamide or dimethylacetamide, an aromatic hydrocarbon such as toluene, benzene or xylene, or a mixture of a dialkylamide and an aromatic hydrocarbon. Alternatively, the inert organic solvent employed may be dimethylsulfoxide, a cyclic ether such as tetrahydrofuran or a mixture thereof. The temperature is from about 15° to about 50°C. With benzyl as group Bz the compound of formula VII has the (R) configuration. Alternatively alkylation may be effected with e.g. 1-bromooctadecane in the presence of sodium hydride (60 % dispersion) and tetrabutyl ammonium iodide. The reaction is conveniently carried out in the presence of an inert, organic solvent, e.g. a cyclic ether such as tetrahydrofuran, at a temperature of from about 0°C to the reflux temperature of the solvent.

**Step 5** involves hydrogenolytic deprotection of the compound of formula VII, preferably, e.g. when Bz is benzyl, by dissolution in a lower alkanol such as methanol or ethanol, or a mixture of a lower alkanol and water (up to about 15 %) and addition of palladium on carbon, if desired with a trace amount of acetic acid, and subjecting the resultant mixture to hydrogen gas under pressure, at a temperature of from about 20° to about 50°C.

In **Step 6** the compound of formula VIII, after dissolution in an inert, organic solvent, e.g. a halogenated hydrocarbon such as methylene chloride or chloroform, or an aromatic hydrocarbon such as benzene or toluene, is reacted with, preferably, 2-chloro-2-oxo-1,3,2-dioxaphospholane in the presence of a tertiary one compound, e.g. a C₁₋₄-trialkylamine such as triethylamine, and optionally a catalyst, e.g. a catalytic amount of 4-dimethylaminopyridine. The reaction is typically carried out at a temperature of from about 20° to about 40°C.

**Step 7** preferably is conducted by reaction with a tertiary alkylamine compound such as trimethylamine. The reaction preferably is conducted in the presence of an inert organic solvent, e.g. a lower alkanol such as methanol or ethanol, an aromatic hydrocarbon such as toluene or benzene, a dialkylamide such as dimethylformamide, or acetonitrile. Although the reaction temperature is not critical, the reaction is typically carried out at a temperature of from about 50° to about 70°C.

Steps 6 and 7 may advantageously be replaced by **Step 6'** involving, in a **first part**, reaction of the compound of formula VIII with phosphorus oxychloride in the presence of an amine base such as pyridine or triethylamine. The reaction is conveniently carried out in the presence of an inert, organic solvent, e.g. a halogenated hydrocarbon such as methylene chloride. The temperature is from about 0° to about 40°C. The **second part** of Step 6' involves reaction of the product produced in the first part with Choline reagent (i.e. ethanaminium,2-hydroxy,N,N,N-trimethyl-4-methylbenzenesulfonate) in the presence of an amine base such as pyridine or triethylamine and a catalytic amount of 4-dimethylaminopyridine. The reaction is conveniently carried out at a temperature of from about 10° to about 40°C.

As regards the preparation of the **enantiomer of the compound of formula I**, this is effected according to the same process and along similar lines, again starting from the compound of formula II in protected form. However, since the configuration at the asymmetrically substituted carbon atom has to be reversed, the sequence of substitution, protection and deprotection steps is different. Thus, the individual reactions are effected e.g. according to the following sequence: Step 1 (hereafter named Step A); Step 4 (hereafter Step B); Step 3 (hereafter Step C); Step 6 (hereafter Step D); and Step 7 (hereafter Step E); whereby above Steps 2 and 5 are not required and Steps 6 and 7 may, as described above, advantageously be replaced by Step 6' (hereafter named Step D').

The preparation of the enantiomer of the compound of formula I can be summarized with the following reaction sequence: 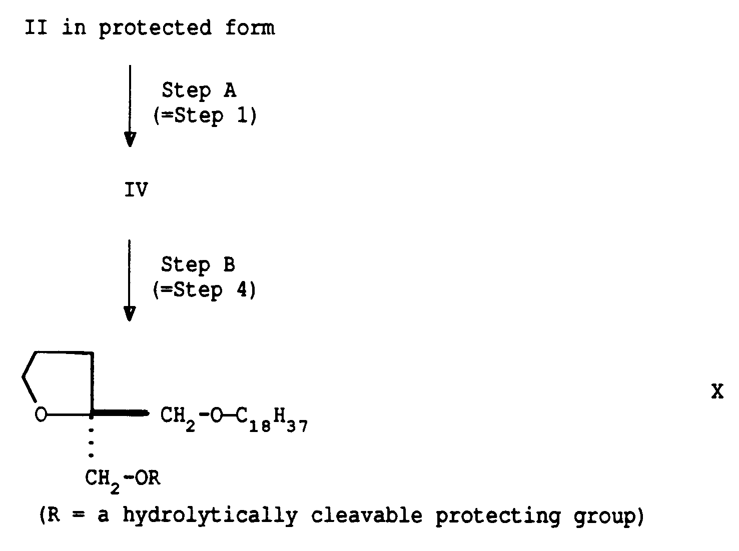 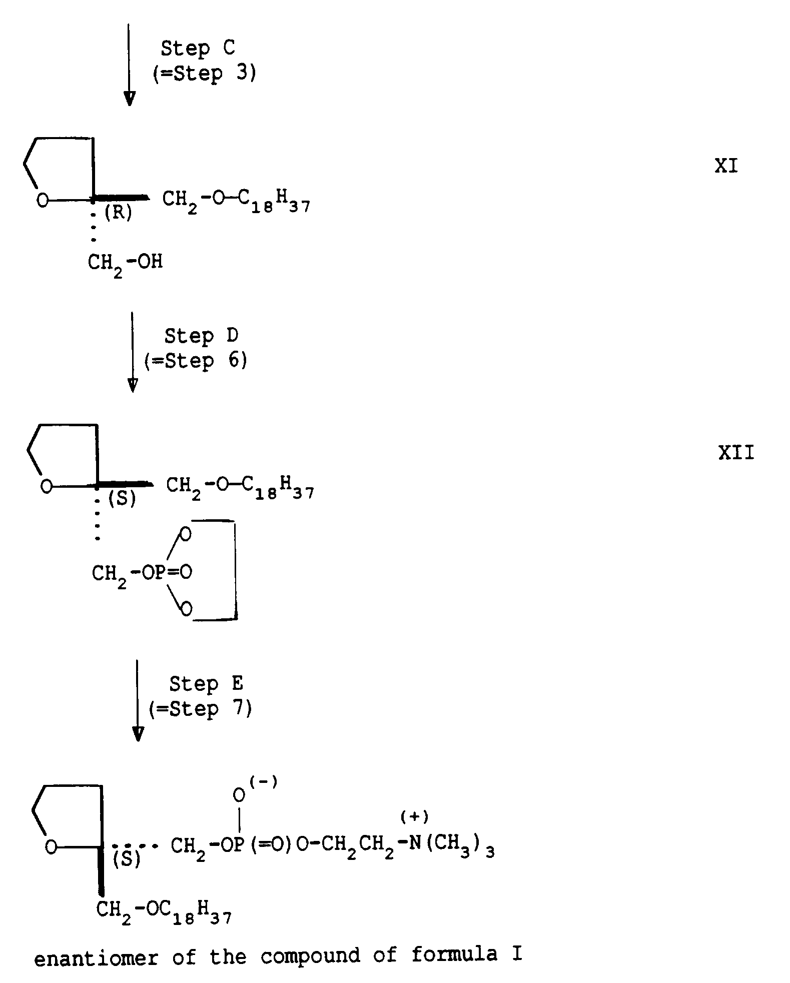
**Steps A, B, C, D** and **E** are effected in analogous manner to Steps 1, 4, 3, 6 and 7 above, respectively; **Step D'** is effected analogously to Step 6' above. The compound of formula X wherein R is tert-butyldiphenylsilyl or trityl has the (S) configuration.

The novel **process of the invention** for the preparation of the **compound of formula II** is effected by adding an appropriate enzyme to a buffered aqueous suspension of compound of formula III. The amount of enzyme added preferably is between about 1 mg and about 40 mg per mmol of compound of formula III.

A suitable enzyme is e.g. the porcine pancreas lipase or a microbial lipase selected from Candida lipolytica lipase, Mucor javanicus lipase and Pseudomonas fluorescens lipase, all of which are known and commercially available. Preferred enzymes are porcine pancreas, Mucor javanicus and Pseudomonas fluorescens lipase. The enzyme preferably is added to a buffered aqueous suspension of the dibutyric ester of formula III, the pH of which has been adjusted to 7 (with either acetic acid or dilute sodium hydroxide). Preferably, the enzyme is added to a buffered suspension of the dibutyric ester in a cosolvent mixture, which mixture comprises water and a n-C₅₋₇-aliphatic hydrocarbon, i.e. pentane, hexane or heptane, in a 1:1 ratio, with the pH of the suspension having been adjusted to 7 prior to the addition of the enzyme.

Alternatively, the dibutyric ester of formula III is added to a buffered aqueous suspension of the enzyme, the pH of which has been adjusted to 7 (with e.g. either acetic acid or dilute sodium hydroxide). Preferably, the dibutyric ester is added to a buffered suspension of the enzyme in a cosolvent mixture, which mixture comprises water and a n-C₅₋₇-aliphatic hydrocarbon, i.e. pentane, hexane or heptane, in a 1:1 ratio, with the pH of the suspension having been adjusted to 7 prior to the addition of the dibutyric ester.

The temperature preferably is of from about 0° to about 30°C.

It is preferred, because of its limited stability, to convert the compound of formula II in free form to a more stable form wherein the hydroxy group is **protected**. Preferred protecting groups are silyl groups such as tert-butyldiphenylsilyl, and bulky aralkyl groups such as triphenylmethyl (trityl).

For **silylation** the free compound of formula II preferably is reacted, e.g. in crude form, with an appropriate silyl derivative such as t-butylchlorodiphenylsilane in the presence of 2 equivalents of imidazole to yield the corresponding protected compound, e.g. the (S)-isomer of formula IIa. Silylation is conveniently carried out in a polar, aprotic solvent, e.g., a dialkylamide such as dimethylformamide or dimethylacetamide, or a lower alkyl nitrile such as acetonitrile, at a temperature of between about 0° and about 30°C.

For **protection with a bulky aralkyl group** the free compound of formula II preferably is reacted with an appropriate, reactive aralkyl derivative such as, for tritylation, triphenylchloromethane, in the presence of an organic base such as triethylamine or pyridine to yield the corresponding protected compound, e.g. the (R)-isomer of formula IIb. The reaction is conveniently carried out in an inert, organic solvent, e.g. a chlorinated, aliphatic hydrocarbon such as methylene chloride, at a temperature of from about -30° to about 30°C.

It will be appreciated that in addition to its use for preparing the compound of formula I and the (S)-enantiomer thereof as described herein, the compound of formula II and its protected forms can be used for preparing further pharmacologically active compounds having an asymmetrically substituted carbon atom adjacent to the oxygen atom in a tetrahydrofuran ring, e.g. the optical isomers of certain anti-tumor compounds disclosed in USP 4'673'672, when it is desired to prepare such compounds in optically pure form.

The compound of formula I and its enantiomer and the compound of formula II in free form or protected form may be isolated from their respective reaction mixtures and purified by conventional techniques such as column chromatography, preparative thin layer chromatography or fractional distillation.

The compound of formula III used as a starting material may be prepared by methods described in the literature, for example by reaction of 2,2-bis(hydroxymethyl)tetrahydrofuran with butyryl chloride.

The following Examples illustrate the invention. All temperatures are in degrees Centigrade.

### Example 1: (R)-2-[(2-Octadecyloxymethyltetrahydrofuran-2-ylmethoxy)-hydroxyphosphinyloxy]-N,N,N-trimethylethanaminium hydroxide inner salt-4-oxide

### [Compound of formula I]

### [Process: Octadecylation and phosphorylcholination of the compound of formula II, with intermediate protection and deprotection]

**a) Step 1:** 48.82 g of **compound of formula IIb** (see Example 5), 3.2 g of potassium carbonate and 500 ml of methanol are stirred at room temperature overnight, after which time the reaction mixture is diluted with 400 ml of ethyl acetate and washed with saturated ammonium chloride solution. The aqueous layer is then separated and extracted three times with ethyl acetate. The ethyl acetate layers are combined and washed two times with saturated sodium chloride solution, dried over sodium sulfate and filtered. The solvent is then removed and the crude product chromatographed on silica gel, first employing an eluent mixture of ethyl acetate and hexane 1:3, and then an eluent mixture of ethyl acetate and hexane 1:1. **(S)-2-Hydroxymethyl-2-trityloxymethyltetrahydrofuran** (compound of formula IV wherein R is trityl) is obtained (M.P. 75.2-77.6°; [α]_{D}²⁵ = - 3.85° in methanol).
**b) Step 2:** A mixture of 9.857 g of sodium hydride (60 % dispersion), 6.961 g of tetrabutylammonium iodide and 205 ml of tetrahydrofuran is cooled to 5°. To the cooled mixture is then added a solution of 54.284 g of the compound prepared in Step 1 above in 205 ml of tetrahydrofuran, and the resultant mixture is warmed to room temperature and stirred for 15 minutes. A solution of 20.7 ml of benzyl bromide in 245 ml of tetrahydrofuran is then added and the reaction mixture is heated to reflux and maintained at this temperature, with stirring, for 2½ hours. The reaction mixture is then cooled to 10° and quenched with 45 ml of isopropyl alcohol. 900 ml of a saturated ammonium chloride solution is then added, and the organic layer separated and washed with saturated sodium chloride solution, dried over sodium sulfate, filtered and concentrated. The crude product is then chromatographed on silica gel employing an eluent mixture of hexane and ethyl acetate 95:5. **(S)-2-benzyloxymethyl-2-trityloxymethyltetrahydrofuran** (compound of formula V wherein R is trityl and Bz is benzyl) is obtained.
**c) Step** **3****:** To a stirred solution of 64.719 g of the compound prepared in Step 2 above and 1600 ml of methylene chloride is added a precooled solution of 240 ml of trifluoroacetic acid in 750 ml of water, and the resultant solution is stirred at room temperature overnight. The organic layer is then separated, washed two times with saturated sodium bicarbonate solution, washed two times with saturated sodium chloride solution, dried over sodium sulfate, filtered and concentrated. The crude product is then chromatographed on silica gel, first employing an eluent mixture of ethyl acetate and hexane 1:2, and then employing an eluent mixture of ethyl acetate and hexane 3:2 to yield **(R)-2-benzyloxymethyl-2-hydroxymethyltetrahydrofuran** (compound of formula VI wherein Bz is benzyl).
**d) Step** **4****:** A mixture of 6.764 g of sodium hydride (60 % dispersion), 4.775 g of tetrabutylammonium iodide and 130 ml of tetrahydrofuran is cooled to 5° under a nitrogen atmosphere. To the cooled mixture is then added a solution of 22.11 g of the compound prepared in Step 3 above in 130 ml of tetrahydrofuran, and the resultant mixture is warmed to room temperature and stirred for 20 minutes. A solution of 39.79 g of 1-bromooctadecane in 155 ml of tetrahydrofuran is added and the reaction mixture is heated to the reflux temperature and stirred overnight. The reaction mixture is then cooled to 5° and quenched with 50 ml of isopropanol, then 500 ml of a saturated ammonium chloride solution is added. The organic layer is then separated and washed two times with saturated sodium chloride solution, dried over sodium sulfate, filtered and concentrated to remove the solvent. The crude product is then chromatographed on silica gel, first employing hexane as the eluent, and then employing an eluent mixture of ethyl acetate and hexane 1:6 to yield **(R)-2-benzyloxymethyl-2-octadecyloxymethyltetrahydrofuran** (compound of formula VII wherein Bz is benzyl).
**e)** **Step 5****:** 11.5 g of 10 % palladium on carbon is added in a Parr bottle to a solution of 22.523 g of the compound prepared in Step 4 above in 250 ml of a 95 % ethanol/water mixture. 10 drops of acetic acid is then added and the mixture is hydrogenated at room temperature under pressure until uptake is complete. The catalyst is then filtered off and the filtrate concentrated in vacuo. The residue is then chromatographed over silica gel employing an eluent mixture of ethyl acetate and hexane 1:3 to yield **(S)-2-hydroxymethyl-2-octadecyloxymethyltetrahydrofuran** (compound of formula VIII).
**f)** **Step 6'****:** To a 500 ml, 4-neck, round bottom flask is added 10.042 g of the compound prepared in Step 5 above and 200 ml of methylene chloride, and the resultant solution is cooled to 3° under a nitrogen atmosphere. To the cooled solution is added 2.57 ml of phosphorus oxychloride, then 4.57 ml of triethylamine is added, dropwise, while the temperature is maintained at below 5°. The resultant mixture is then stirred at room temperature for 20 hours and cooled to 5°. To the cooled mixture is then added 22.8 ml of pyridine, 0.296 g of 4-dimethylaminopyridine and 13.897 g of Choline reagent. The reaction mixture is then stirred at room temperature for 72 hours, the solids are removed by filtration, and the mixture is concentrated to remove the solvents. The resultant residue is then dissolved in a mixture of 205 ml of tetrahydrofuran, 48 ml of water and 25 ml of pyridine, and the resultant solution is heated to the reflux temperature and maintained there for 5 hours. The solution is then cooled to room temperature, filtered through a column of 685 g of Amberlite MB-3 ion exchange resin, and eluted with a mixture of 10 % water in tetrahydrofuran. The fractions containing the desired product are then collected, combined and concentrated and the resultant residue is cooled to 0°. 170 ml of acetone is then added, dropwise, and the resultant suspension is stirred at 0° for 30 minutes, after which time a yellow waxy solid is collected by filtration. The solid is then chromatographed on silica gel, first employing an eluent mixture of methylene chloride, methanol and water 79:19:2, and then employing an eluent mixture of methylene chloride, methanol and water 10:5:1. The fractions are then combined, concentrated to a volume of 40 ml and cooled to 5°. 160 ml of acetone is then added, dropwise, and a white precipitate forms. The mixture is then stirred at 5° for 30 minutes and the solid collected by filtration and then dissolved in 250 ml of absolute ethanol. After 200 ml of the solvent is removed, the remainder is added, dropwise, to 260 ml of acetone. 1.2 ml of water is then added and the mixture cooled to 5° and maintained at this temperature overnight. The resultant solid is collected by filtration, washed with cold acetone and dried on a high vacuum pump at room temperature to yield the **title compound** (M.P. 193°; [α]_{D}²⁵ = + 1.9° in ethanol).

### Example 2: (S)-2-[(2-Octadecyloxymethyltetrahydrofuran-2-ylmethoxy)-hydroxyphosphinyloxy)-N,N,N-trimethylethanaminium hydroxide inner salt-4-oxide

### [Enantiomer of the compound of formula I]

### [Process: as for Example 1]

**a) Step A:** See Example 1, Step 1.
**b)** **Step B****:** Analogously to Example 1, Step 4, but using the compound of Step A above in place of the compound of Step 3, **(S)-2-octadecyloxymethyl-2-trityloxymethyltetrahydrofuran** (compound of formula X wherein R is trityl) is obtained.
**c)** **Step C****:** Analogously to Example 1, Step 3, but using the compound of Step B above in place of the compound of Step 2, **(R)-2-hydroxymethyl-2-octadecyloxymethyltetrahydrofuran** (compound of formula XI) is obtained.
**d)** **Step D'****:** Analogously to Example 1, Step 6', but using the compound of Step C above in place of the compound of Step 5, the **title compound** is obtained (M.P. 188°; [α]_{D}²⁵ = - 1.9° in ethanol).

### Example 3: (R)-2,2-Bis(hydroxymethyl)tetrahydrofuran monobutyrate

### [Compound of formula II]

### [Process: Enantiospecific monohydrolysis]

**a)** **Using PPL****:** 1.36 g (5 mmol) of **2,2-bis(hydroxymethyl)tetrahydrofuran dibutyrate** (compound of formula III) is suspended in a buffered mixture comprising 50 ml of water and 50 ml of hexane, and the pH of the resultant suspension is adjusted to 7 by adding acetic acid and/or 2 N sodium hydroxide. To the resultant buffered suspension is added, with stirring, 100 mg of porcine pancreas lipase (PPL) (from Sigma Chem. Corp.; 42 U/mg), which results in an immediate reaction. The reaction is allowed to proceed while the pH is continually adjusted to 7 by the addition of 2 N sodium hydroxide. After the reaction slows down (about 45 minutes), the phases are separated and the aqueous phase is extracted three times with ethyl acetate. The organic phases are then combined, dried over magnesium sulfate, filtered and concentrated in vacuo to yield the crude product. Flash chromatography on silica gel employing a mixture of hexane and ethyl acetate 1:1 as the eluent and Kugelrohr distillation yields the **title compound** (oil; yield 89 %; chemical purity 99 %; [α]_{D}²⁵ = + 19.5° in toluene).
**b)** **Using PPL****:** Into a 3-liter flask equipped with a stirrer, pH meter probe and auto-buret is added 1 liter of pH 7 buffer, 1 liter of hexane and 2.2 g of lipase PPL, then the pH of the resultant suspension is adjusted to 7 by adding acetic acid. To the resultant suspension is added with stirring 27.2 g of 2,2-bis(hydroxymethyl)tetrahydrofuran, dibutyrate (diluted with minimum amount of hexane). The reaction is allowed to proceed while the pH is continually adjusted to 7 by the addition of 2 N sodium hydroxide. After the reaction is about 98 % complete, the reaction mixture is poured into 1 l of ethyl acetate and extracted. The organic layer is separated, dried over sodium sulfate, filtered through silica gel, and evaporated to yield the **title compound** in crude form (oil; chemical purity > 99 %; [α]_{D}²⁵ = + 19.5° in toluene; B.P. 125° at 0.5 mmHg employing bulb to bulb distillation).
**c)** **Using LMJ****:** Analogously to a) above but using 19 mg of microbial lipase Mucor javanicus (LMJ) (from Fluka Chemical Co.; 5 U/g) in place of PPL and allowing the reaction to proceed for 5½ hours in place of 45 minutes, the **title compound** is obtained (oil; yield 64 %; [α]_{D}²⁵ = + 14.3° in toluene).

The starting material is obtained as follows:

Into a two-neck, round bottom flask (equipped with an addition funnel, a reflux condenser and an outlet to an HCl trap) containing 101.6 g of 2,2-bis(hydroxymethyl)tetrahydrofuran in 800 ml of dichloromethane, 176 ml of butyryl chloride is added (slightly exothermic) over a period of 40 minutes. The resultant solution (hydrogen chloride gas is quenched), is stirred for 21 hours, followed by the addition of 1200 ml of an aqueous sodium bicarbonate solution over a period of 30 minutes. The phases are separated and the aqueous phase is extracted three times with dichloromethane. The organic phases are combined, dried over magnesium sulfate, filtered and concentrated in vacuo. The resultant yellow residue is distilled to yield **2,2-bis(hydroxymethyl)tetrahydrofuran dibutyrate** (colorless liquid; B.P. 127-128° at 0.4 mmHg).

### Example 4: (S)-2-Diphenylsilyloxymethyl-2-hydroxymethyltetrahydrofuran butyrate

### [Compound of formula IIa]

### [Process: Protection of the compound of formula II]

6.06 g of the crude form of the compound of Example 3a) is dissolved in 75 ml of dimethylformamide and to the solution is added 4.08 g of imidazole and 12.38 g of t-butylchlorodiphenylsilane. The resultant solution is then stirred at room temperature until no starting material is detectable (about 3 to 12 hours). 75 ml of water is then added and the resultant mixture extracted four times with diethyl ether. The organic phases are then combined, washed with 100 ml of water, dried over magnesium sulfate, filtered and concentrated in vacuo. Medium pressure liquid chromatography on silica gel employing a mixture of hexane and ethyl acetate 9:1 as the eluent yields the **title compound** (oil).

### Example 5: (R)-2-Hydroxymethyl-2-triphenylmethoxymethyltetrahydrofuran butyrate

### [Compound of formula IIb]

### [Process: Protection of the compound of formula II]

21.5 g of the compound prepared in Example 3b) above, is dissolved in 400 ml of methylene chloride and the resultant solution is cooled to -30°. To the cooled solution is added 10.51 ml of pyridine and a solution of 33.454 g of triphenylchloromethane in 100 ml of methylene chloride, while maintaining the temperature at -30°. The reaction mixture is then allowed to warm slowly overnight to room temperature, then it is diluted with 400 ml of ethyl acetate, washed two times with 2 N hydrochloric acid, and washed two times with saturated sodium chloride solution. The organic layer is then dried over sodium sulfate, filtered and the solvents removed to yield the **title compound** in crude form.

Further, it has been found that the compound of formula I and its enantiomer possess surprising and advantageous pharmacological properties.

As indicated above, the corresponding racemate is known from e.g. USP 4'673'672, Example 5, as an anti-tumor agent. It has now been found that the anti-tumor properties reside essentially in the (S)-isomer, the (R)-isomer, i.e. the compound of formula I, being ineffective in that indication.

Even more surprisingly, it has been found that the compound of formula I, on the other hand, i.e. the (R)-isomer, is indicated for use in multiple sclerosis. This novel activity resides essentially in the (R)-isomer while the (S)-isomer is ineffective. This activity had not been disclosed anywhere for the racemate on the priority date of the present invention for this use.

The present invention thus involves two enantiomeric compounds which each possesses a beneficial pharmacological activity not shared by the other isomer. This finding emphasizes the desirability and benefit, alluded to above in general terms, of providing both isomers in pure form.

The activity of the **compound of formula I** in treating **multiple sclerosis** can be demonstrated employing the following test methods:

### 1. Experimental allergic encephalomyelitis (EAE test) in the rat; preventive treatment:

### [Levine et al., Am. J. Path. 47 (1965) 61; McFarlin et al., J. Immunol. 113 (1974) 712; Borel, Transplant. & Clin. Immunol. 13 (1981) 3]:

Male Wistar rats are injected in the hind paws with 0.1 ml of a mixture of bovine spinal cord and complete Freund's adjuvant. The test compound is administered at dosages of from 5 to 50 mg/kg/day p.o. 5 days a week, commencing on the day of sensitization and continuing for 2 weeks. Onset of EAE in control groups receiving no medication generally commences between 9 to 16 days after sensitization and is marked by symptoms of paralysis in the hind limbs and tail. Test animals are examined daily for symptoms of the disease and disease occurrence is scored according to their severity. Total paralysis means complete involvement of both hind legs and tail. The test animals are kept under observation for a total period of 50 days.

On administration of the compound of formula I at the above-indicated dosages, a substantial reduction of occurrence of clinical symptoms is observed over the test period in comparison with occurrence in control groups receiving placebo.

### 2. Experimental allergic encephalomyelitis (EEA test) in the rat; therapeutic treatment:

Testing is carried out analogously to that described above with the exception that the administration of the test compound commences on day 7 after sensitization (i.e. immediately prior to appearance of disease symptoms) at dosages of from 5 to 50 mg/kg/day p.o. either daily or every second day and continuing for one week. During the testing period the animals are examined daily for symptoms of the disease and scored as in the previous test method.

On administration of the compound of formula I at the above dosages, a substantial reduction of appearance of EAE disease symptoms is observed over the test period in comparison with appearance in control groups receiving placebo.

The following results were obtained when the racemate, i.e., the compound of Example 5 of USP 4'673'672, and the compound of formula I and its enantiomer were administered at 25 mg/kg/day:

| EAE test | | | | |
|---|---|---|---|---|
| Compound | Preventive treatment | | Therapeutic treatment | |
| | Animals with total paralysis) | | Animals with EAE) | |
| | No./total¹⁾ | % | No./total¹⁾ | % |
| Control | 33/40 | 83 | 40/40 | 100 |
| Example 5 of USP 4'673'672 | 3/7 | 43 | 2/7 | 29 |
| Compound of formula I | 5/14 | 36 | | |
| Enantiomer of the compound of formula I | 12/14 | 86 | | |

| | | | | |
|---|---|---|---|---|
| ¹⁾ Number of animals with total paralysis or, respectively, symptoms of disease per total number of rats treated | | | | |

As can be seen from the above results, the enantiomer of the compound of formula I appears to be devoid of any usefulness in treating multiple sclerosis upon administration of 25 mg/kg/day in the above method. Moreover, it can be seen that the compound of formula I is clearly more effective in treating multiple sclerosis than is the racemic mixture, i.e. the compound of Example 5 of USP 4'673'672.

### 3. Chronic relapsing experimental allergic encephalomyelitis (CR-EAE test) in the rat; therapeutic treatment:

Testing is effected analogously to that described above with the exception that the antigenic mixture consists of guinea-pig spinal cord emulsified in Freund's adjuvant enriched with Mycobacterium tuberculosis. The test compound is administered at dosages of from 5 to 50 mg/kg/day p.o. for 16 days, starting on day 16 which corresponds to the beginning of the first spontaneous remission. During the test period the animals are examined daily for symptoms of disease and scored as previously described.

Both the compound of formula I and the compound of Example 5 of USP 4'673'672 are effective in inhibiting the appearance of mild to severe clinical symptoms for the period of treatment. However, even more remarkably, withdrawal of the drugs on day 32 is not followed by an exacerbated relapse in the treated animals: treatment prevents recurrence of further relapses upon discontinuation of the drug, i.e. the rats are cured. It also becomes evident that the compound of formula I is effective to fully protect all animals both during treatment and following discontinuation of drug. The enantiomer of the compound of formula I shows a much less effective protective efficacy during treatment since several animals show signs of EAE. The control group enters a strong relapse during the treatment period and after day 35 several rats go into a third and protracted relapse phase.

### 4. Delayed-type hypersensitivity (DTH test) in the mouse:

Suppression of DTH mediated by cloned helper T cells is tested with mice injected subcutaneously with cloned helper T cells together with the relevant antigen, namely sheep erythrocytes, in one hind footpad and with the same clone plus an unrelated antigen as control in the other footpad. The drugs are administered orally twice, first one day before and again at the time of the cell injection. After 24 h the increase in footpad thickness is measured. Footpad swelling is indicated as percentage compared with the control group (placebo).

The following results were obtained:

| DTH test | | |
|---|---|---|
| Compound | Suppression of DTH (%) at a dosage of | |
| | 2 x 40 mg/kg | 2 x 80 mg/kg |
| Control | 0 | 0 |
| Example 5 of USP 4'673'672 | 31 | 88 |
| Compound of formula I | 73 | 88 |
| Enantiomer of the compound of formula I | 16 | 40 |

Suppression of the DTH reaction is observed with both the compound of formula I and Example 5 of USP 4'673'672. In contrast, the enantiomer of the compound of formula I shows a comparatively very weak inhibitory effect at both dosages.

The precise dosage of the compound of formula I to be employed in treating multiple sclerosis depends upon several factors including the host, the nature and the severity of the condition being treated and the mode of administration. However, in general, satisfactory inhibition of the symptoms of multiple sclerosis is achieved when the compound is administered orally at a daily dosage of between about 0.5 mg/kg and about 30 mg/kg body weight, preferably between about 1 mg/kg and about 20 mg/kg. The total daily dosage is normally between about 100 mg and about 600 mg. A preferred total daily dosage is between 100 mg and 300 mg.

The compound of formula I may be combined with one or more pharmaceutically acceptable carriers and, optionally, one or more conventional pharmaceutical adjuvants and administered orally in the form of tablets, dispersible powders, granules, capsules, elixirs, suspensions and the like. The compositions may be prepared by conventional means.

The activity of the **enantiomer** of the compound of formula I in treating **tumors** can be demonstrated employing the following test methods:

### 1. Tumor cell cytotoxicity test (TCC test):

In flat bottom microtiter plates (Nunc Roskieide, Denmark) are placed Abelson 8.1 lymphoma, YAC-1, L1210 or P815 tumor cells in Dulbecco modified Eagle's medium (DMEM) + 10 % fetal calf serum and the tumor cell-containing plates are incubated with 1, 3 and 5 »g of the test compound for a period of 6 to 72 hours. The number of viable tumor cells present in the Abelson 8.1, YAC-1, L1210 and P815 assays is determined by measuring the alkaline phosphatase in the following manner: the tumor cell plates are centrifuged (500 x g) for ten minutes and the supernatant flicked off. Without further washing, 100 »l of buffer containing 20 »l of diethanolamine, 2 »M of MgCl₂·6H₂O, 2.5 »M of p-nitrophenylphosphate and 10 mg Triton X-100 are added. The samples are incubated for 60 minutes at room temperature and the enzymatic reaction is terminated by the addition of 100 »l of 0.5 N NaOH. The absorbance is then measured at 405 nM using a Titertek Multiskan apparatus.

At an incubation period of 72 hours the following results were obtained:

| TCC test | | | | | |
|---|---|---|---|---|---|
| Compound | Concentration (»g/ml) | Inhibition with tumor cells (%) | | | |
| | | Abelson 8.1 | YAC-1 | L1210 | P815 |
| Example 5 of USP 4'673'672 | 1 | 68 | 19 | 0 | 40 |
| | 3 | 97 | 74 | 80 | 87 |
| | 5 | 98 | 91 | 91 | 95 |
| Enantiomer of the compound of formula I | 1 | 91 | 40 | 45 | 84 |
| | 3 | 98 | 78 | 92 | 93 |
| | 5 | 98 | 91 | 97 | 97 |
| Compound of formula I | 1 | 29 | 8 | 16 | 32 |
| | 3 | 93 | 86 | 76 | 80 |
| | 5 | 95 | 96 | 87 | 97 |

As can be seen from the above results, the (S)-enantiomer of the compound of formula I is clearly more effective in treating tumors than is the racemic mixture, i.e. the compound of Example 5 of USP 4'673'672, and the compound of formula I. More particularly, the (S)-isomer is more effective in inhibiting different types of tumors than is either the racemic mixture or the (R)-isomer, especially at lower concentrations.

### 2. Influence on cytotoxicity of ET-18-OCH₃ test (IC-ET test) :

Bone marrow cell macrophages (10⁵/well) obtained from [BALB/CX57/BL₆]F1 mice are incubated with 10 »g of (±)-1-octadecyl-2-methoxy-3-phosphorylcholine (ET-18-OCH₃) for 24 hours in flat bottom microtiter plates (Nunc Roskieide, Denmark), then they are centrifuged and washed once. Abelson 8.1 lymphoma, YAC-1, L1210 or P815 tumor cells in DMEM + 10 % fetal calf serum and 1 »g, 3 »g and 5 »g of the test compound are then added to the plates. With the cytotoxicity of ET-18-OCH₃ (10 »g) alone set at 100 %, the inhibition or enhancement of the cytotoxic effect, as measured by an alkaline phosphatase assay, is determined and values recorded after 72 hours for the test substance.

The following results were obtained:

| IC-ET test | | | | | |
|---|---|---|---|---|---|
| Compound | Concentration (»g/ml) | Enhancement with tumor cells (%) | | | |
| | | Abelson 8.1 | YAC-1 | L1210 | P815 |
| Example 5 of USP 4'673'672 | 1 | 99.6 | 84 | 90.6 | 96.6 |
| | 3 | 99.8 | 97.5 | 98.4 | 99.3 |
| | 5 | 99.9 | 98.8 | 99.1 | 99.4 |
| Enantiomer of the compound of formula I | 1 | 99.5 | 89 | 96.6 | 97.6 |
| | 3 | 99.7 | 97.7 | 99.4 | 98.9 |
| | 5 | 99.8 | 99.1 | 99.8 | 99.7 |
| Compound of formula I | 1 | 99.2 | 89 | 94.7 | 96.9 |
| | 3 | 99.7 | 98.4 | 98.6 | 98.2 |
| | 5 | 99.6 | 99.1 | 98.9 | 98.7 |

As can be seen from the above results, the enantiomer of the compound of formula I is more effective in enhancing the cytotoxic effect of ET-18-OCH₃ in different types of tumors than are the racemic mixture, i.e. the compound of Example 5 of USP 4'673'672, and the compound of formula I.

### 3. In vivo Meth A fibrosarcoma test in the mouse:

Meth A fibrosarcoma cells are induced in BALB/C mice by administering methylcholanthrene according to the procedure of Old et al. (Ann. N.Y. Acad. Sci. 101 [1962] 80). These tumor cells are harvested from the peritoneal cavity 10 to 12 days after administration of methylcholanthrene. Ten CBF₁ mice of 10-12 week age are each implanted with 7.3 x 10⁶ Meth A sarcoma cells to serve as control. A second group of ten CBF₁ mice are each implanted with 7.3 x 10⁶ Meth A sarcoma cells and on day one after implant each mouse is treated p.o. with from 5 »g to 50 »g of the test compound per day for a total of twenty or twenty-seven days. Tumor growth and survivors are assayed on days 7, 14, 21 and 28 after tumor implantation.

The following results were obtained:

| In vivo Met A fibrosarcoma test in the mouse | | | | | | |
|---|---|---|---|---|---|---|
| Compound | Concentration (»g)mouse) | Tumor volume (% of control) | | | | Survivors (tumor free) |
| | | day 7 | day 14 | day 21 | day 28 | |
| Example 5 of USP 4'673'672 | 5 | 82 | 47 | 20 | 32 | 3/10 |
| | 50 | 76 | 38 | 14 | 21 | 6/10 |
| Enantiomer of the compound of formula I | 5 | 66 | 19 | 6 | 11 | 8/10 |
| | 50 | 70 | 28 | 8 | 13 | 8/10 |
| Compound of formula I | 5 | 90 | 52 | 21 | 37 | 2/10 |
| | 50 | 85 | 53 | 21 | 32 | 3/10 |

As can be seen from the above results, the enantiomer of the compound of formula I is much more effective in treating tumors than is the racemic mixture, i.e. the compound of Example 5 of USP 4'673'672, or the (R) compound of formula I, especially at the lower dose. Moreover, whereas none of the control group animals survived, 8 out of 10 animals which were administered 5 »g of the (S)-isomer survived.

In view of the fact that the enantiomer of the compound of formula I is more effective in treating tumors than is the racemic mixture, i.e., Example 5 of USP 4'673'672, it can be concluded that pharmaceutical compositions comprising the enantiomer of the compound of formula I as the active ingredient offer a therapeutic advantage over pharmaceutical compositions comprising the racemic mixture.

As with the multiple sclerosis use regarding the compound of formula I, the precise dosage of the enantiomer of the compound of formula I to be employed for inhibiting tumors, e.g. various lymphomas, sarcomas, myelomas and leukemias, depends upon several factors including the host, the nature and the severity of the condition being treated, the mode of administration and the particular compound employed. However, in general, satisfactory inhibition of tumors is achieved when the compound is administered enterally, preferably orally, or parenterally, e.g. intravenously, at a daily dosage from about 1 mg/kg to about 100 mg/kg, preferably from about 2 mg/kg to about 50 mg/kg body weight. The total daily dosage is normally between about 50 mg and about 1000 mg, preferably between about 100 mg and about 700 mg. A typical oral daily dosage is 400 mg, or 10 mg/kg intravenously over a 24 hour period. A typical dosage unit for oral administration two to four times a day in the inhibition of tumors may contain from about 75 mg to about 300 mg of the compound. Preferred oral dosage units for inhibition of tumors contain from about 75 mg to about 250 mg, especially from about 100 mg to about 200 mg of the compound. The enantiomer of the compound of formula I may be combined with one or more pharmaceutically acceptable carriers and administered orally in the form of tablets, dispersible powders, granules, capsules, elixirs and suspensions.

The compound of formula I and, respectively, its enantiomer may be formulated into such pharmaceutical compositions containing an amount of said optically active compound that is effective in treating multiple sclerosis or, respectively, tumors, such compositions being in unit dosage forms and comprising a solid pharmaceutically acceptable carrier.

Tablets and capsules containing the ingredients indicated below may be prepared by conventional techniques and are useful in treating multiple sclerosis or, respectively, in inhibiting tumors:

| Ingredients | Tablet | Capsule |
|---|---|---|
| | weight (mg) | |
| Compound of formula I or its enantiomer | 150 | 150 |
| Tragacanth | 10 | - |
| Lactose (spray-dried) | 197.5 | 250 |
| Corn starch | 25 | - |
| Talcum | 15 | - |
| Magnesium stearate | 2.5 | - |
| total: | 4̅0̅0̅ | 4̅0̅0̅ |

The preferred pharmaceutical compositions from the standpoint of preparation and ease of administration are solid compositions, particularly liquid or hard-filled capsules and tablets containing from about 100 mg to about 300 mg of compound of formula I or, respectively, from about 100 mg to about 200 mg of its enantiomer.

The invention thus further includes a pharmaceutical composition comprising the compound of formula I or, respectively, its enantiomer, together with at least one pharmaceutically acceptable carrier or diluent.

It further includes a process for the preparation of a pharmaceutical composition comprising mixing the compound of formula I or its enantiomer together with at least one pharmaceutically acceptable carrier or diluent.

It further includes the compound of formula I and its enantiomer for use as a pharmaceutical, namely as an agent in the preventive or therapeutic treatment of multiple sclerosis as regards the compound of formula I and as an anti-tumor agent as regards its enantiomer.

## Claims (Claims for the following Contracting State(s): AT, BE, CH, DE, DK, FR, GB, IT, LI, LU, NL, SE)

1. The compound of formula I 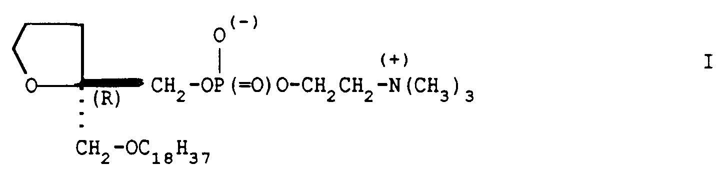 namely (R)-2-[(2-octadecyloxymethyltetrahydrofuran-2-ylmethoxy)hydroxyphosphinyloxy]-N,N,N-trimethylethanaminium hydroxide inner salt-4-oxide or its enantiomer.

2. The compound of formula I as defined in claim 1.

3. The enantiomer of the compound of formula I as defined in claim 1.

4. A process for the preparation of the compound of formula I as defined in claim 1 comprising octadecylating and phosphorylcholinating (R)-2,2-bis(hydroxymethyl)tetrahydrofuran monobutyrate of formula II 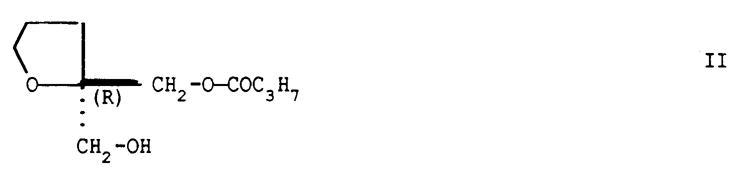 with intermediate protection and deprotection steps,
according to the following reaction sequence: 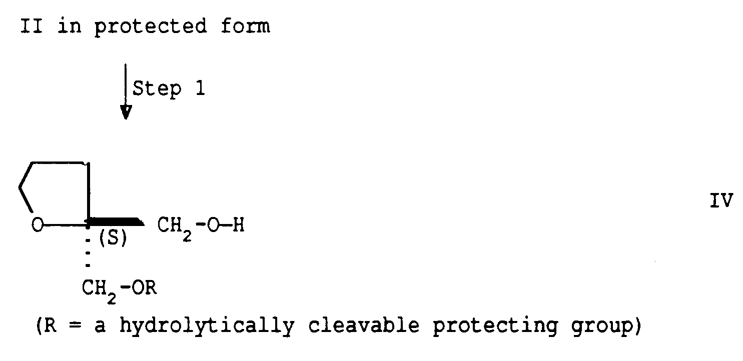 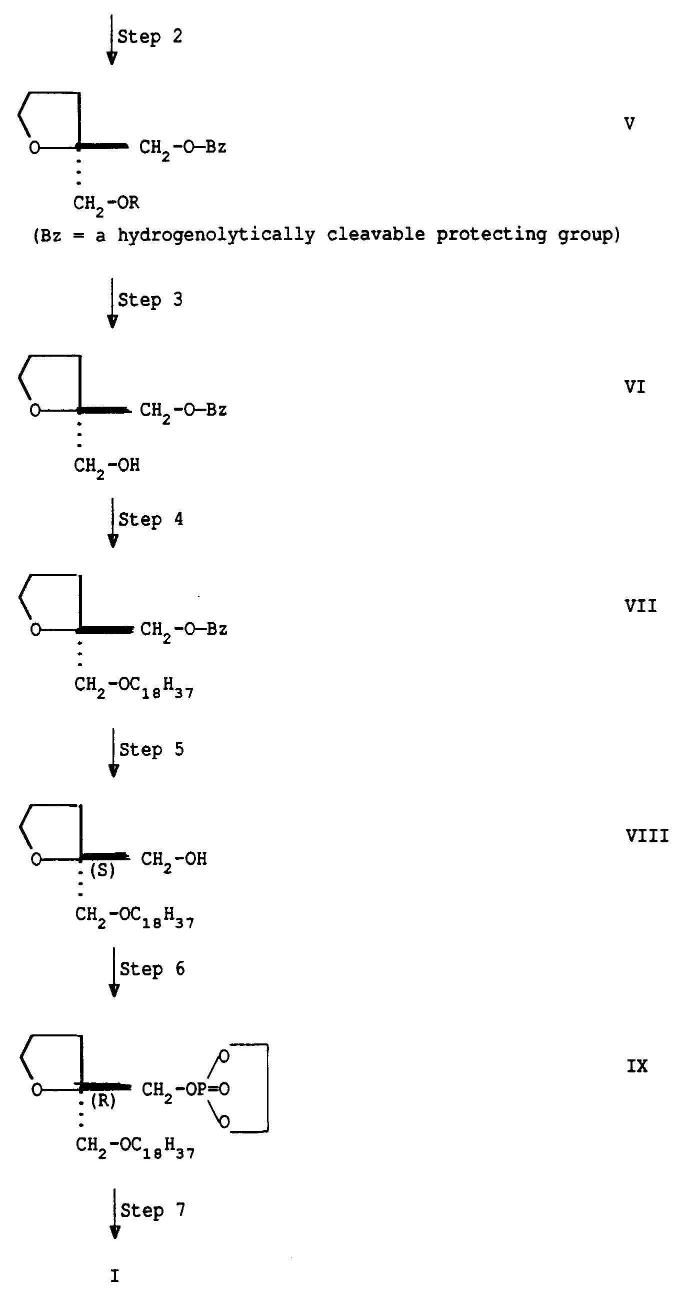 whereby the following reactions are effected:
- Step 1: alcoholic hydrolysis
- Step 2: protection with a hydrogenolytically cleavable protecting group
- Step 3: hydrolytic deprotection
- Step 4: alkylation
- Step 5: hydrogenolytic deprotection
- Step 6: acylation with 2-chloro-2-oxo-1,3,2-dioxaphospholane
- Step 7: amination with trimethylamine.

5. A process for the preparation of the enantiomer of the compound of formula I, according to the following reaction sequence: 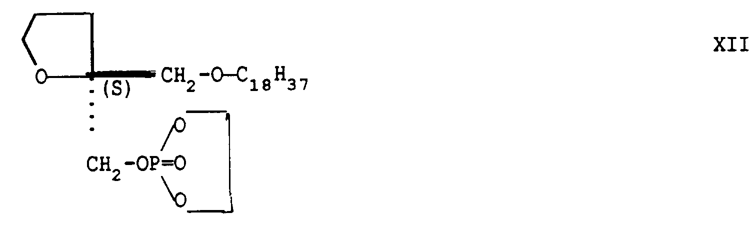 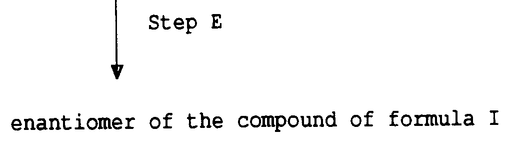 whereby the following reactions are effected:
- Step A: alcoholic hydrolysis
- Step B: alkylation
- Step C: hydrolytic deprotection
- Step D: acylation with 2-chloro-2-oxo-1,3,2-dioxaphospholane
- Step E: amination with trimethylamine.

6. A pharmaceutical composition comprising the compound of formula I as defined in claim 1 or its enantiomer together with at least one pharmaceutically acceptable carrier or diluent.

7. The compound of formula I as defined in claim 1 or its enantiomer for use as a pharmaceutical.

8. The compound of formula I as defined in claim 1 for use in the treatment of multiple sclerosis.

9. (R)-2,2-bis(hydroxymethyl)tetrahydrofuran monobutyrate of formula II 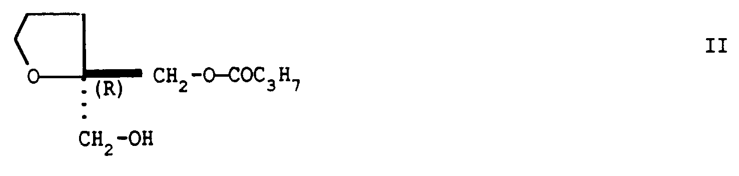 in free form or in protected form.

10. A process for the preparation of a compound according to claim 9 comprising enantiospecifically monohydrolysing 2,2-bis(hydroxymethyl)tetrahydrofuran dibutyrate of formula III 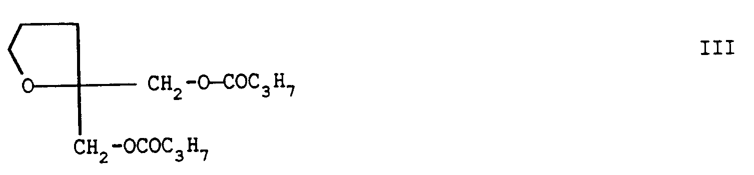 and optionally converting the resultant compound of formula II in free form to a protected form thereof.

11. The compound of formula VIII as defined in claim 4.

12. The compound of formula XI as defined in claim 5.

13. Use of a compound according to claim 9, 11 or 12 as an intermediate in the preparation of the compounds of claim 1.

## Claims (Claims for the following Contracting State(s): ES, GR)

1. A process for the preparation of the compound of formula I 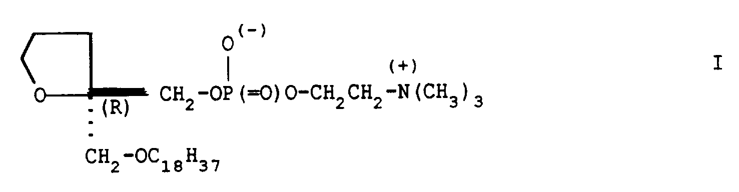 namely (R)-2-[(2-octadecyloxymethyltetrahydrofuran-2-ylmethoxy)hydroxyphosphinyloxy]-N,N,N-trimethylethanaminium hydroxide inner salt-4-oxide
comprising octadecylating and phosphorylcholinating (R)-2,2-bis(hydroxymethyl)tetrahydrofuran monobutyrate of formula II 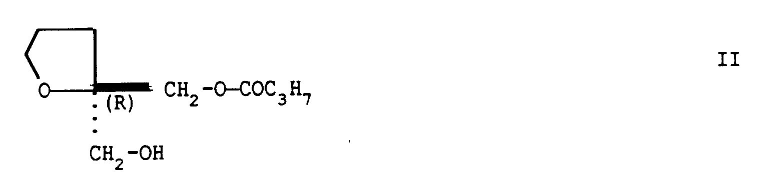 with intermediate protection and deprotection steps,
according to the following reaction sequence: 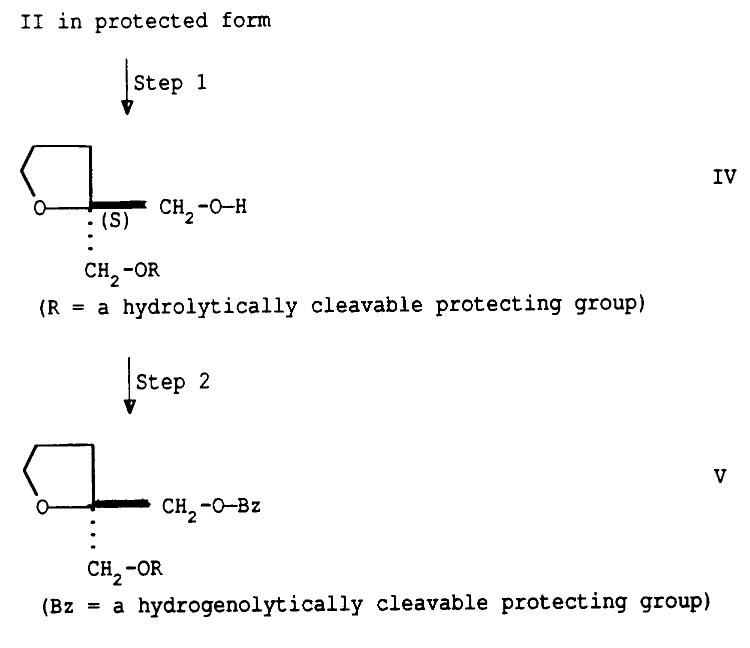 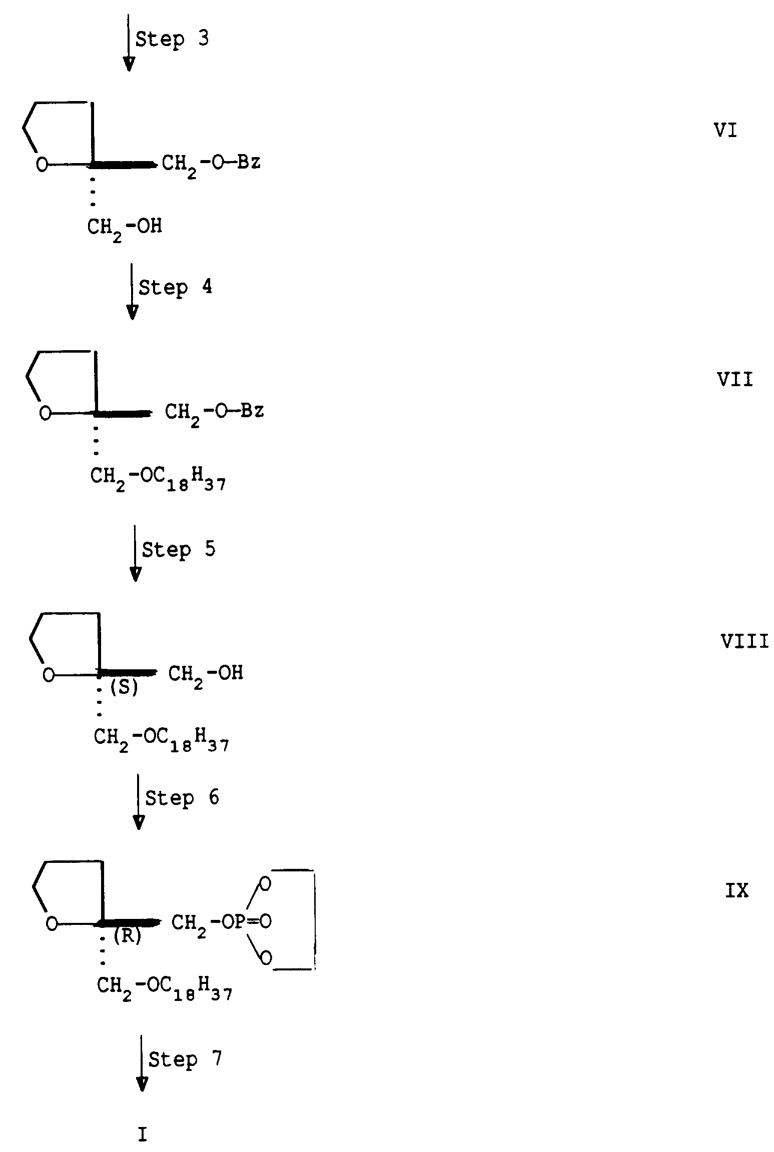 whereby the following reactions are effected:
- Step 1: alcoholic hydrolysis
- Step 2: protection with a hydrogenolytically cleavable protecting group
- Step 3: hydrolytic deprotection
- Step 4: alkylation
- Step 5: hydrogenolytic deprotection
- Step 6: acylation with 2-chloro-2-oxo-1,3,2-dioxaphospholane
- Step 7: amination with trimethylamine.

2. A process for the preparation of the enantiomer of the compound of formula I, according to the following reaction sequence: 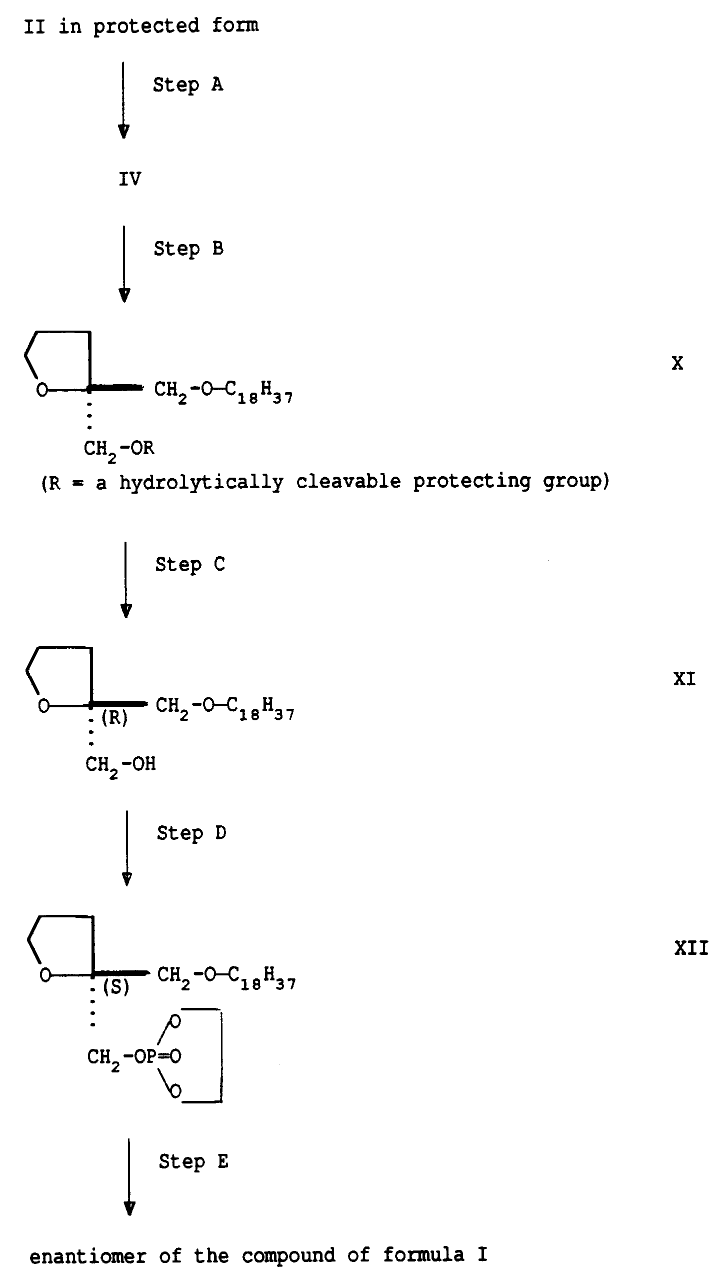 whereby the following reactions are effected:
- Step A: alcoholic hydrolysis
- Step B: alkylation
- Step C: hydrolytic deprotection
- Step D: acylation with 2-chloro-2-oxo-1,3,2-dioxaphospholane
- Step E: amination with trimethylamine.

3. A process according to claim 1 or 2 wherein the compound of formula II is in protected form and is the compound of formula IIa

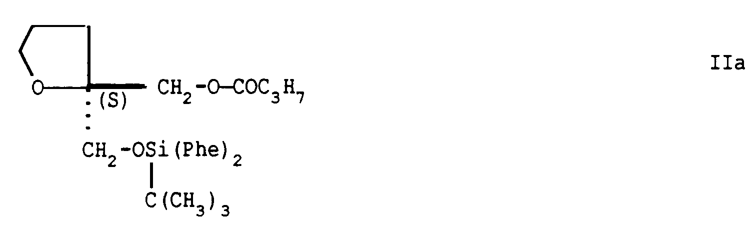

4. A process according to claim 1 or 2 wherein the compound of formula II is in protected form and is the compound of formula IIb

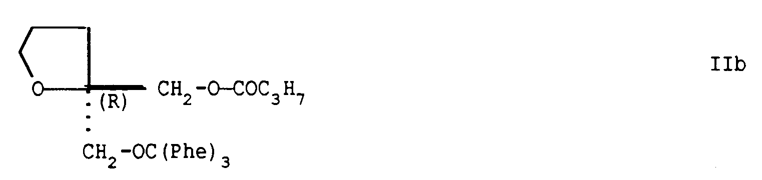

5. A process for the preparation of (R)-2,2-bis(hydroxymethyl)tetrahydrofuran monobutyrate of formula II 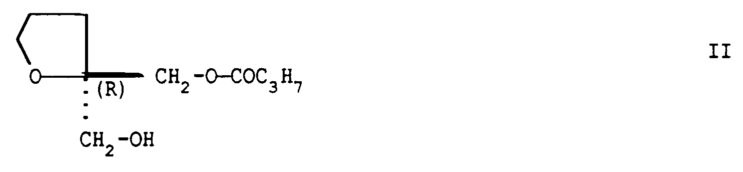 in free form or in protected form, comprising enantiospecifically monohydrolysing 2,2-bis(hydroxymethyl)tetrahydrofuran dibutyrate of formula III 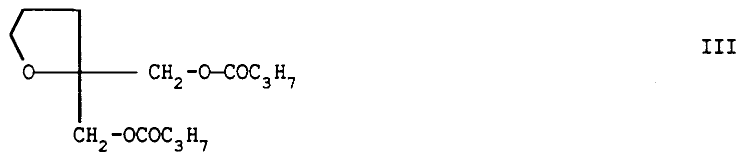 and optionally converting the resultant compound of formula II in free form to a protected form thereof.

6. A process according to claim 5 comprising enzymatic hydrolysis.

7. A process according to claim 5 comprising hydrolysis with a lipase.

8. A process for the preparation of a pharmaceutical composition comprising mixing the compound of formula I as defined in claim 1 or its enantiomer together with at least one pharmaceutically acceptable carrier or diluent.

9. A process for the preparation of the compound of formula VIII as defined in claim 1 comprising the reaction sequence defined in claim 1 with the omission of Steps 6 and 7.

10. A process for the preparation of the compound of formula XI as defined in claim 2 comprising the reaction sequence defined in claim 2 with the omission of Steps D and E.

## Patentansprüche (Patentansprüche für folgende(n) Vertragsstaat(en): AT, BE, CH, DE, DK, FR, GB, IT, LI, LU, NL, SE)

1. Verbindung der Formel I 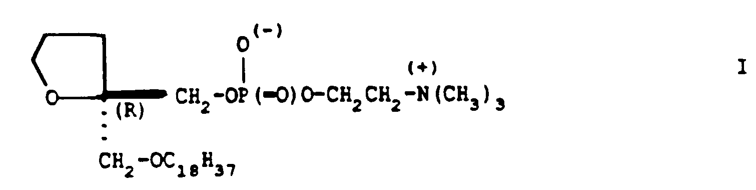 nämlich das innere Salz (R)-2-[(2-Octadecyloxymethyltetrahydrofuran-2-ylmethoxy)hydroxyphosphinyloxy]-N,N,N-trimethylethanaminiumhydroxid-4-oxid oder das Enantiomer davon.

2. Verbindung der Formel I, wie in Anspruch 1 definiert.

3. Enantiomer der Verbindung der Formel I, wie in Anspruch 1 definiert.

4. Verfahren zur Herstellung der Verbindung der Formel I, wie in Anspruch 1 definiert, umfassend, daß man das (R)-2,2-Bis(hydroxymethyl)tetrahydrofuranmonobutyrat der Formel II 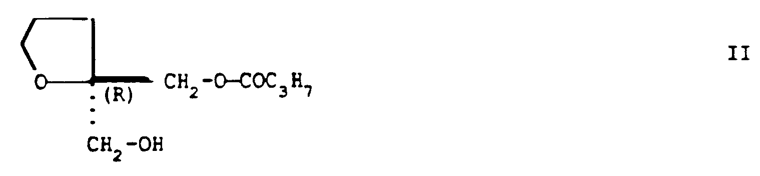 octadecyliert und phosphorylcholiniert, wobei Stufen zur Einführung von Schutzgruppen und Abspaltung von Schutzgruppen zwischengeschaltet sind, gemäß der folgenden Reaktionssequenz: 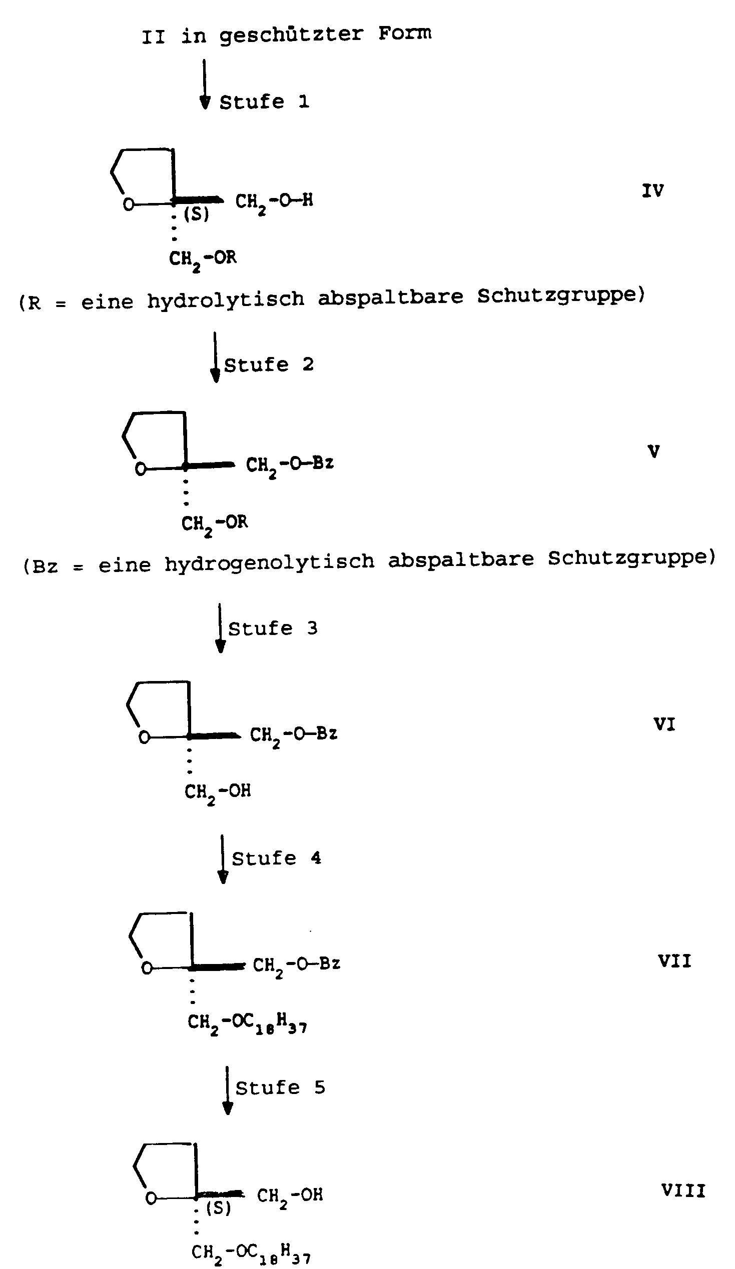 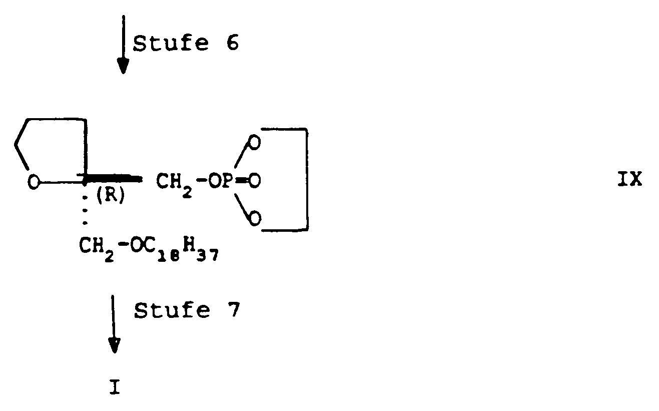 worin die folgenden Reaktionen durchgeführt werden:
- Stufe 1: alkoholische Hydrolyse
- Stufe 2: Schutz mit einer hydrogenolytisch abspaltbaren Schutzgruppe
- Stufe 3: hydrolytische Abspaltung der Schutzgruppen
- Stufe 4: Alkylierung
- Stufe 5: hydrogenolytische Abspaltung der Schutzgruppen
- Stufe 6: Acylierung mit 2-Chlor-2-oxo-1,3,2-dioxaphospholan
- Stufe 7: Aminierung mit Trimethylamin.

5. Verfahren zur Herstellung des Enantiomers der Verbindung der Formel I gemäß der folgenden Reaktionssequenz: 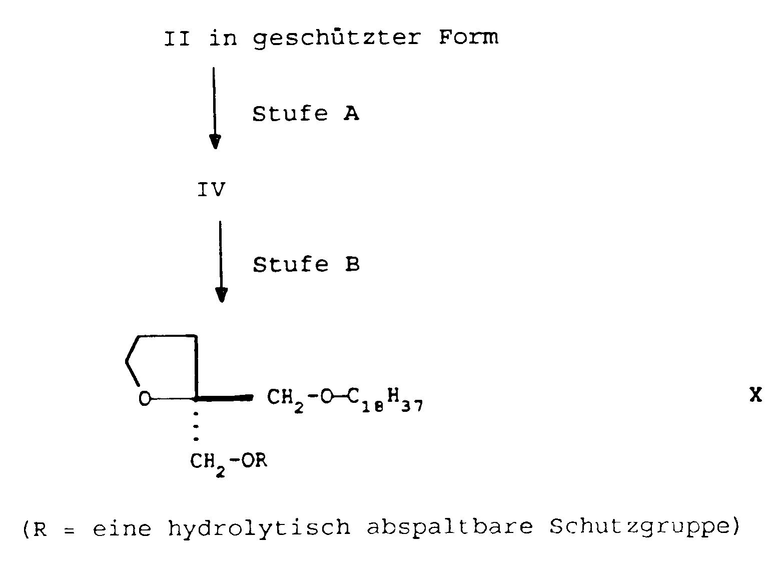 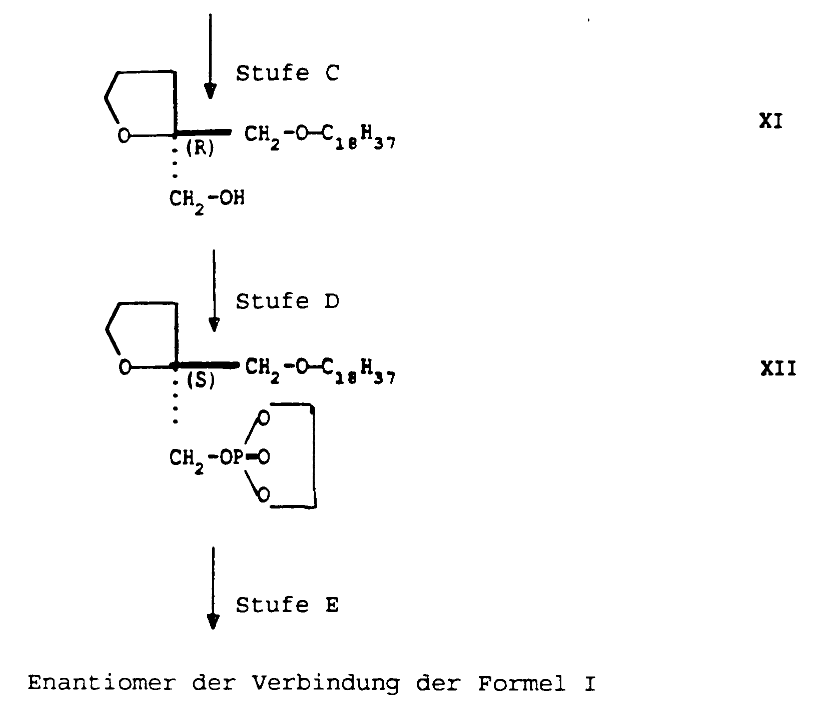 wobei die folgenden Reaktionen durchgeführt werden:
- Stufe A: alkoholische Hydrolyse
- Stufe B: Alkylierung
- Stufe C: hydrolytische Abspaltung der Schutzgruppen
- Stufe D: Acylierung mit 2-Chlor-2-oxo-1,3,2-dioxaphospholan
- Stufe E: Aminierung mit Trimethylamin.

6. Pharmazeutische Zusammensetzung umfassend die Verbindung der Formel I, wie in Anspruch 1 definiert oder das Enantiomer zusammen mit mindestens einem pharmazeutisch annehmbaren Träger oder Verdünnungsmittel.

7. Verbindung der Formel I, wie in Anspruch 1 definiert, oder das Enantiomer zur Verwendung als Pharmazeutikum.

8. Verbindung der Formel I, wie in Anspruch 1 definiert, zur Verwendung zur Behandlung von multipler Sklerose.

9. (R)-2,2-Bis(hydroxymethyl)tetrahydrofuranmonobutyrat der Formel II 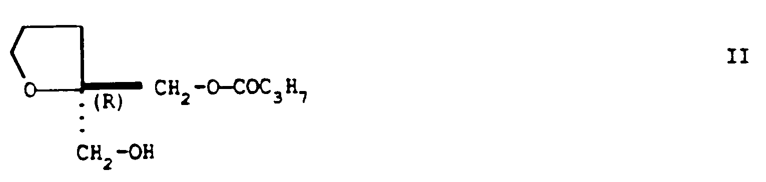 in freier oder geschützter Form.

10. Verfahren zur Herstellung einer Verbindung nach Anspruch 9 umfassend, daß man 2,2-Bis(hydroxymethyl)tetrahydrofurandibutyrat der Formel III 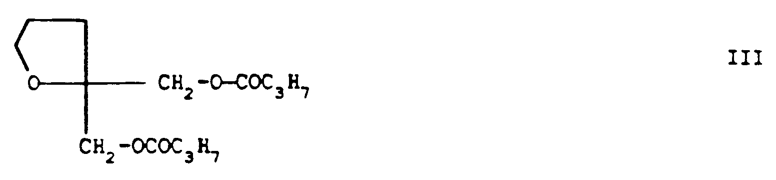 enantiospezifisch monohydrolysiert und gegebenenfalls die entstehende Verbindung der Formel II in die freie Form oder eine geschützte Form umwandelt.

11. Verbindung der Formel VIII, wie in Anspruch 4 definiert.

12. Verbindung der Formel XI, wie in Anspruch 5 definiert.

13. Verwendung einer Verbindung nach Anspruch 9, 11 oder 12 als Zwischenprodukt zur Herstellung der Verbindungen von Anspruch 1.

## Patentansprüche (Patentansprüche für folgende(n) Vertragsstaat(en): ES, GR)

1. Verfahren zur Herstellung der Verbindung der Formel I 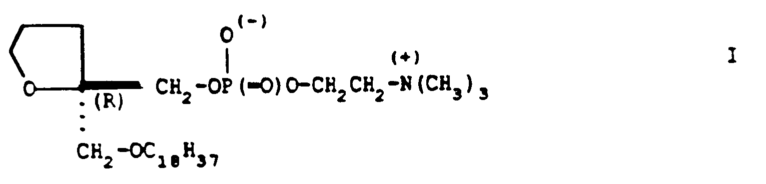 nämlich dem inneren Salz (R)-2-[(2-Octadecyloxymethyltetrahydrofuran-2-ylmethoxy)hydroxyphosphinyloxy]-N,N,N-trimethylethanaminiumhydroxid-4-oxid umfassend, daß man das (R)-2,2-Bis(hydroxymethyl)tetrahydrofuranmonobutyrat der Formel II 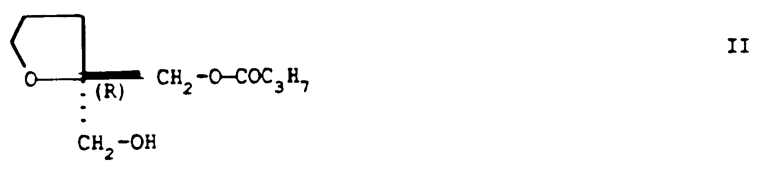 octadecyliert und phosphorylcholiniert, wobei Stufen zur Einführung von Schutzgruppen und Abspaltung von Schutzgruppen zwischengeschaltet sind, gemäß der folgenden Reaktionssequenz: 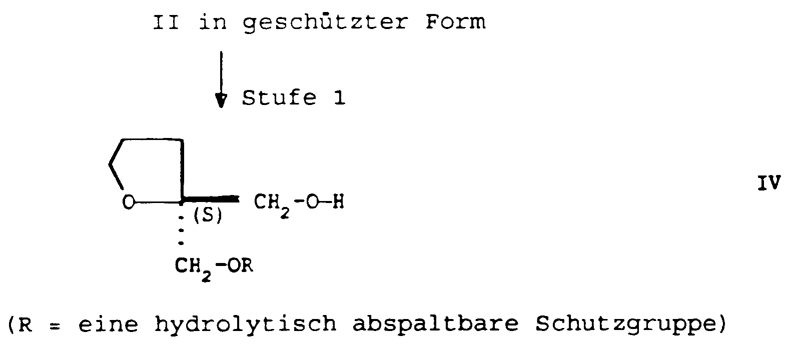 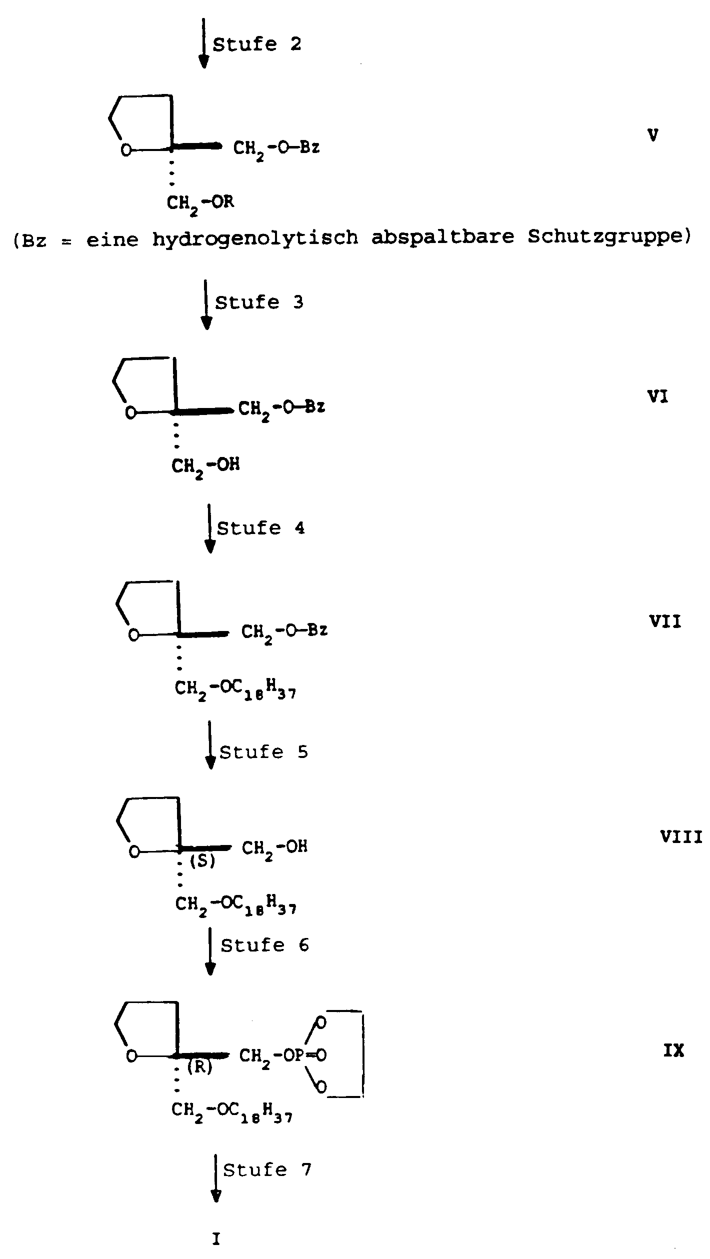 worin die folgenden Reaktionen durchgeführt werden:
- Stufe 1: alkoholische Hydrolyse
- Stufe 2: Schutz mit einer hydrogenolytisch abspaltbaren Schutzgruppe
- Stufe 3: hydrolytische Abspaltung der Schutzgruppen
- Stufe 4: Alkylierung
- Stufe 5: hydrogenolytische Abspaltung der Schutzgruppen
- Stufe 6: Acylierung mit 2-Chlor-2-oxo-1,3,2-dioxaphospholan
- Stufe 7: Aminierung mit Trimethylamin.

2. Verfahren zur Herstellung des Enantiomers der Verbindung der Formel I gemäß der folgenden Reaktionssequenz: 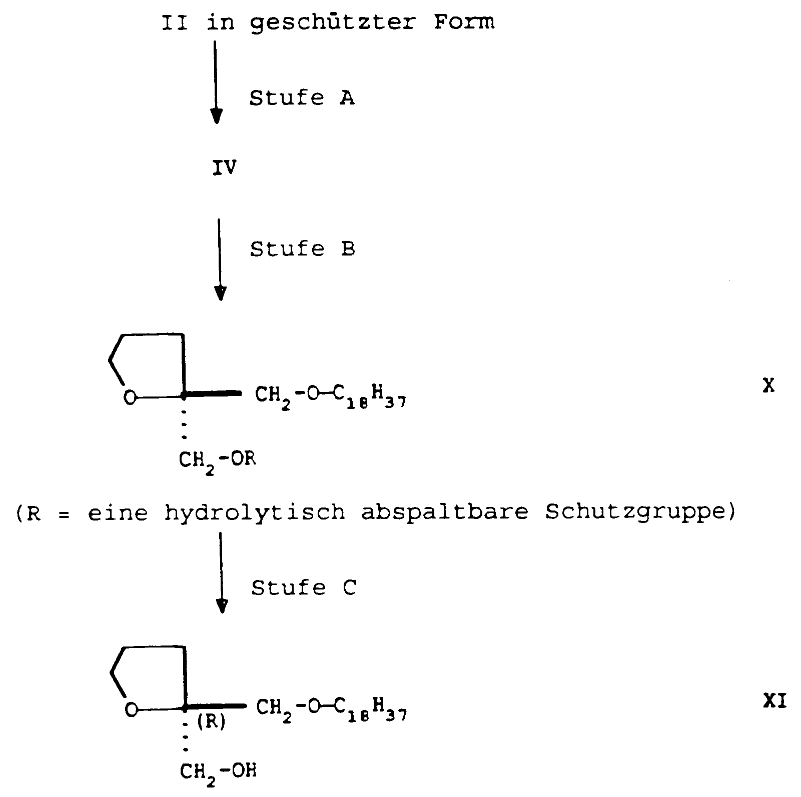 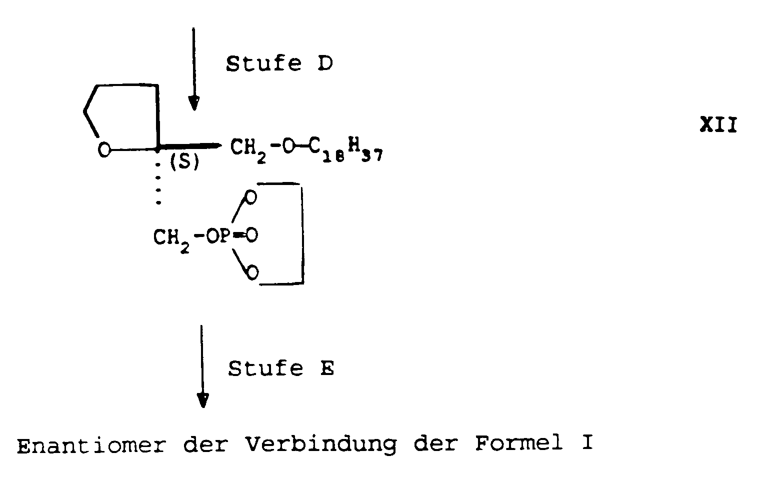 wobei die folgenden Reaktionen durchgeführt werden:
- Stufe A: alkoholische Hydrolyse
- Stufe B: Alkylierung
- Stufe C: hydrolytische Abspaltung der Schutzgruppen
- Stufe D: Acylierung mit 2-Chlor-2-oxo-1,3,2-dioxaphospholan
- Stufe E: Aminierung mit Trimethylamin.

3. Verfahren nach Anspruch 1 oder 2, worin die Verbindung der Formel II in geschützter Form ist und die Verbindung der Formel IIa ist:

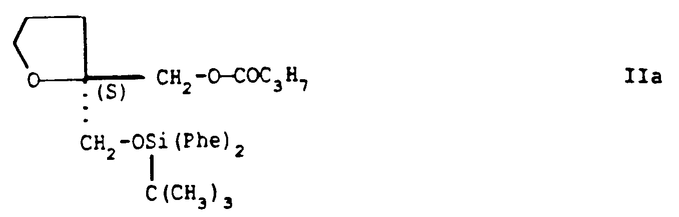

4. Verfahren nach Anspruch 1 oder 2, worin die Verbindung der Formel II in geschützter Form ist und die Verbindung der Formel IIb ist:

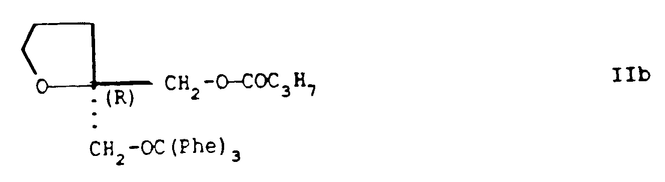

5. Verfahren zur Herstellung von (R)-2,2-Bis(hydroxymethyl)tetrahydrofuranmonobutyrat der Formel II 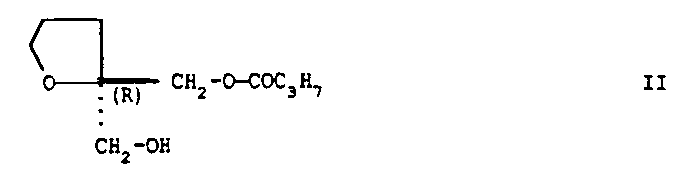 in freier oder geschützter Form, umfassend daß man 2,2-Bis(hydroxymethyl)tetrahydrofurandibutyrat der Formel III 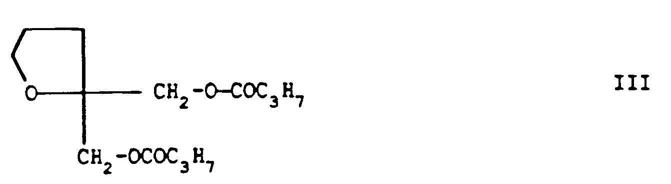 enantiospezifisch monohydrolysiert und gegebenenfalls die entstehende Verbindung der Formel II in freier Form in eine geschützte Form umwandelt.

6. Verfahren nach Anspruch 5 umfassend die enzymatische Hydrolyse.

7. Verfahren nach Anspruch 5, umfassend die Hydrolyse mit einer Lipase.

8. Verfahren zur Herstellung einer pharmazeutischen Zusammensetzung umfassend, daß man die Verbindung der Formel I, wie in Anspruch 1 definiert, oder ein Enantiomer davon mit mindestens einem pharmazeutisch annehmbaren Träger oder Verdünnungsmittel vermischt.

9. Verfahren zur Herstellung der Verbindung der Formel VIII, wie in Anspruch 1 definiert, umfassend die Reaktionssequenz, die in Anspruch 1 definiert ist, wobei die Stufen 6 und 7 ausgelassen werden.

10. Verfahren zur Herstellung der Verbindung der Formel XI, wie in Anspruch 2 definiert, umfassend die Reaktionssequenz, wie in Anspruch 2 definiert, wobei die Stufen D und E ausgelassen werden.

## Revendications (Revendications pour l'(les) Etat(s) contractant(s) suivant(s): AT, BE, CH, DE, DK, FR, GB, IT, LI, LU, NL, SE)

1. Le composé de formule I 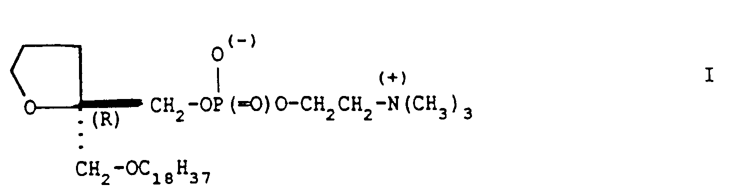 à savoir le 4-oxyde du sel interne de l'hydroxyde de (R)-2-[(2-octadécyloxyméthyltétrahydrofuran-2-ylméthoxy)hydroxyphosphinyloxy]-N,N,N-triméthyléthanaminium ou son énantiomère.

2. Le composé de formule I tel que défini à la revendication 1.

3. L'énantiomère du composé de formule I tel que défini à la revendication 1.

4. Un procédé de préparation du composé de formule I tel que défini à la revendication 1, comprenant l'octadécylation et la phosphorylcholination du monobutyrate de (R)-2,2-bis(hydroxyméthyl)tétrahydrofuranne de formule II 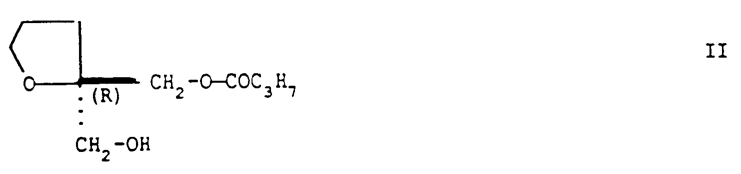 avec les étapes intermédiaires de protection et de déprotection,
selon le schéma réactionnel suivant: 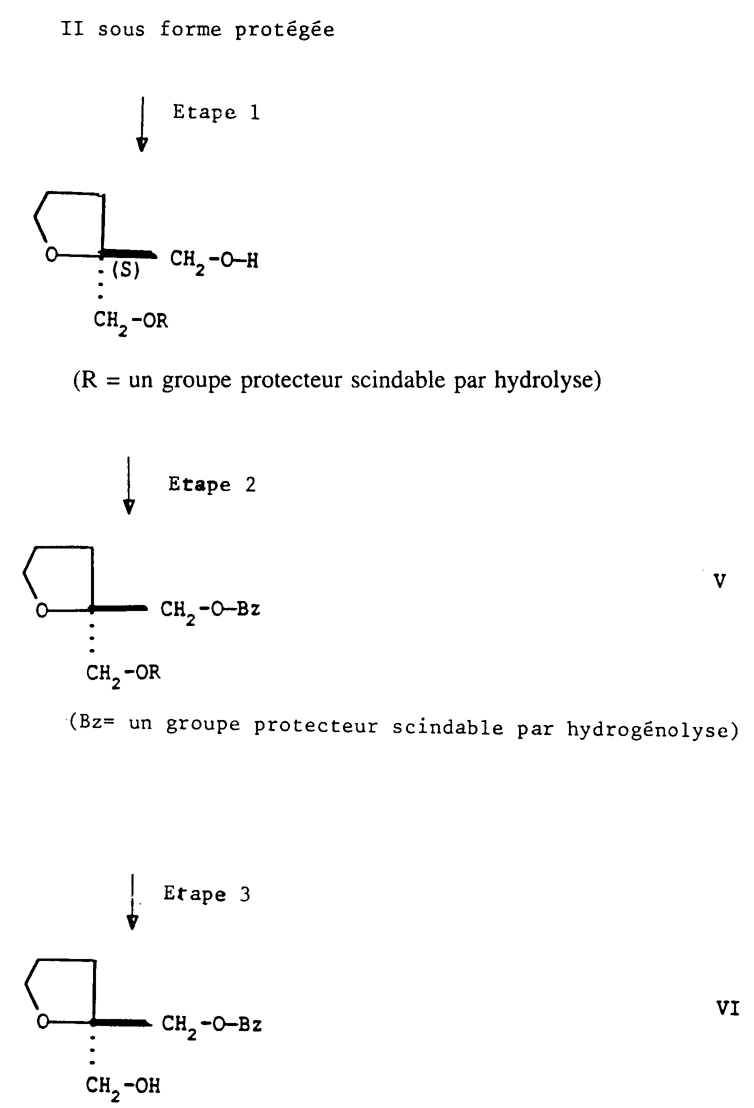 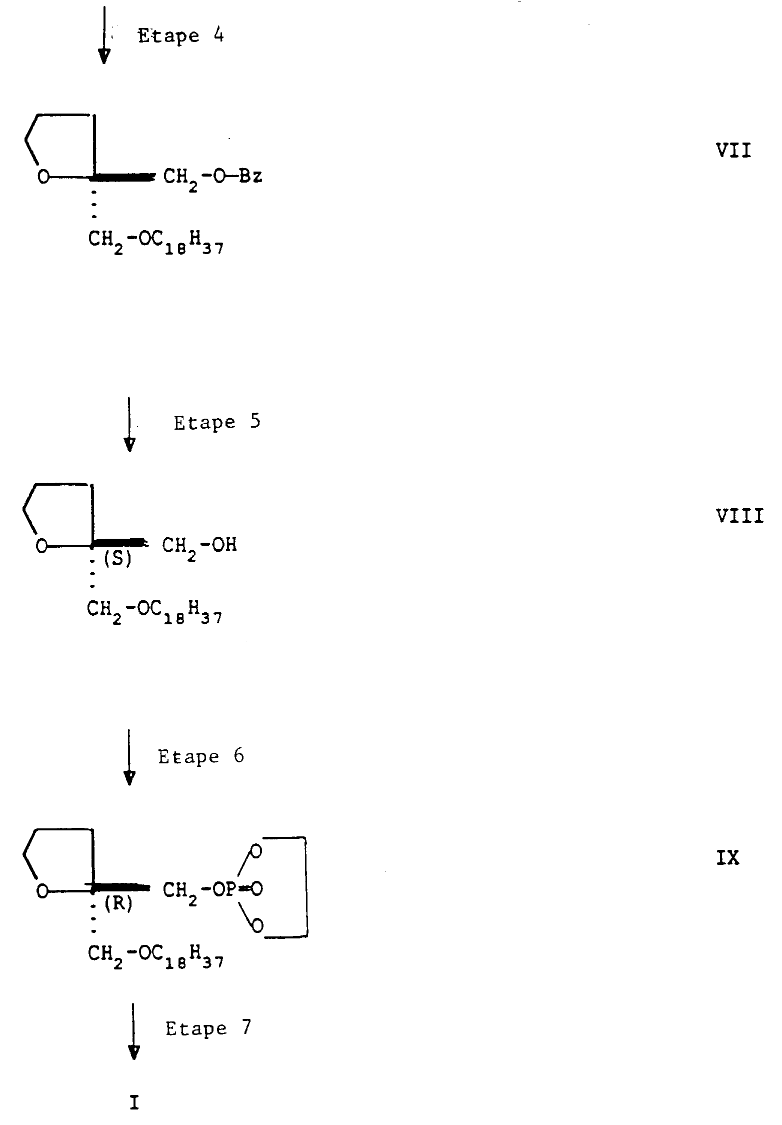 les réactions suivantes étant effectuées:
- Etape 1: hydrolyse alcoolique,
- Etape 2: protection avec un groupe protecteur scindable par hydrogénolyse,
- Etape 3: déprotection hydrolytique,
- Etape 4: alkylation,
- Etape 5: déprotection hydrogénolytique,
- Etape 6: acylation avec le 2-chloro-2-oxo-1,3,2-dioxaphospholane,
- Etape 7: amination avec de la triméthylamine.

5. Un procédé de préparation de l'énantiomère du composé de formule I, selon le schéma réactionnel suivant: 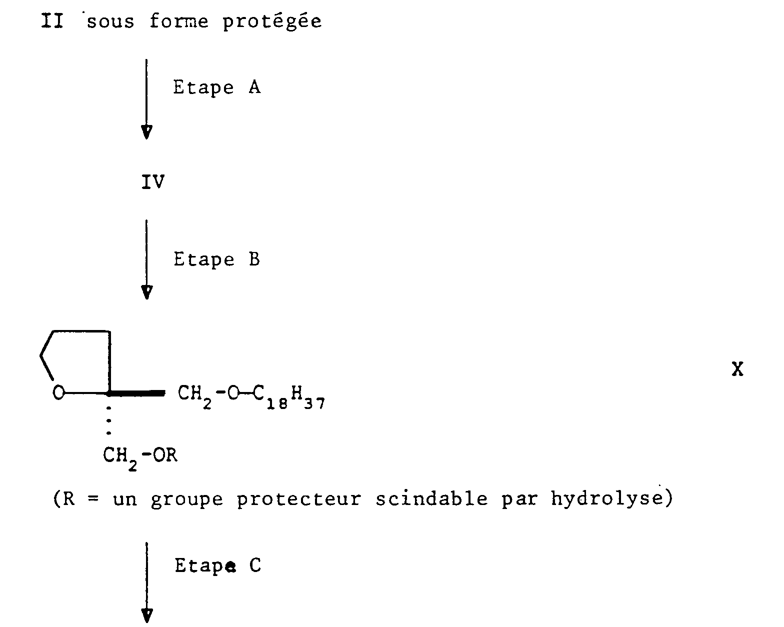 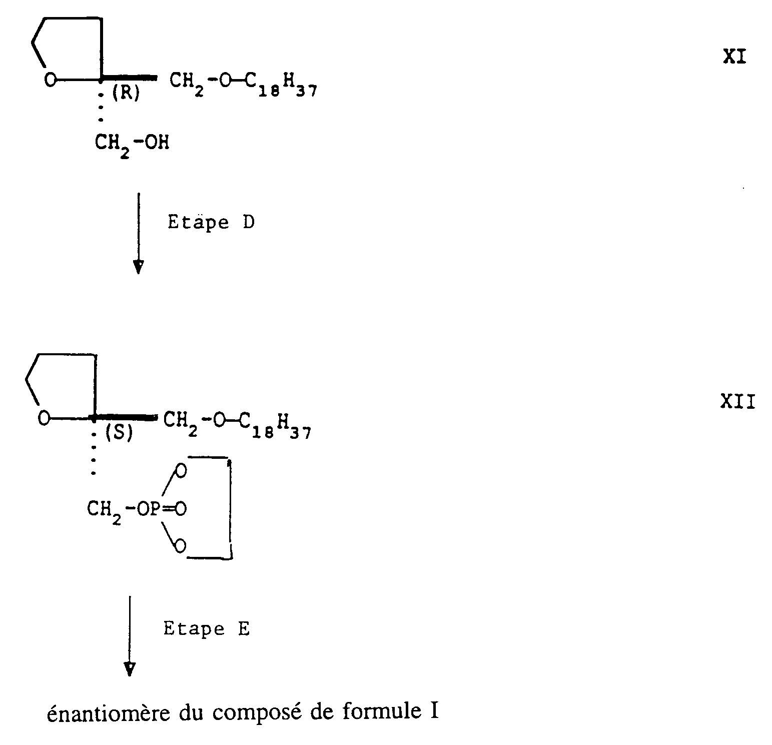 les réactions suivantes étant effectuées:
- Etape A: hydrolyse alcoolique,
- Etape B: alkylation,
- Etape C: déprotection hydrolytique,
- Etape D: acylation avec le 2-chloro-2-oxo-1,3,2,-dioxaphospholane,
- Etape E: amination avec de la triméthylamine.

6. Une composition pharmaceutique comprenant le composé de formule I tel que défini à la revendication 1 ou son énantiomère et au moins un véhicule ou diluant pharmaceutiquement acceptable.

7. Le composé de formule I tel que défini à la revendication 1 ou son énantiomère pour une utilisation comme médicament.

8. Le composé de formule I tel que défini à la revendication 1 pour une utilisation dans le traitement de la sclérose en plaques.

9. Le monobutyrate de (R)-2,2-bis(hydroxyméthyl)tétrahydrofuranne de formule II 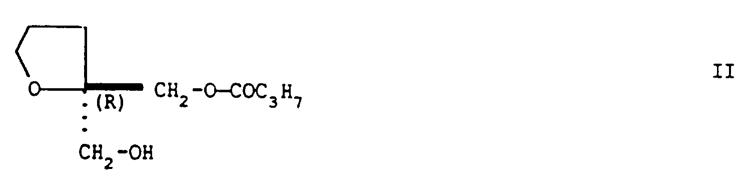 sous forme libre ou sous forme protégée.

10. Un procédé de préparation d'un composé selon la revendication 9, comprenant la monohydrolyse de manière énantio-spécifique du dibutyrate de 2,2-bis(hydroxyméthyl)-tétrahydrofuranne de formule III 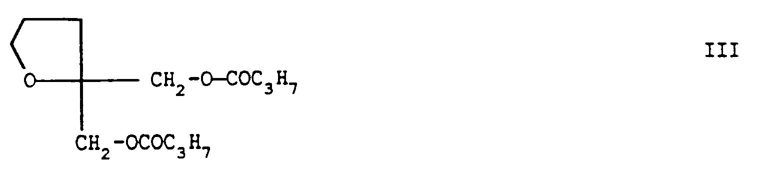 et éventuellement la transformation du composé résultant de formule II sous forme libre en forme protégée.

11. Le composé de formule VIII tel que défini à la revendication 4.

12. Le composé de formule XI tel que défini à la revendication 5.

13. L'utilisation d'un composé selon la revendication 9, 11 ou 12 comme produit intermédiaire pour la préparation des composés selon la revendication 1.

## Revendications (Revendications pour l'(les) Etat(s) contractant(s) suivant(s): ES, GR)

1. Un procédé de préparation du composé de formule I 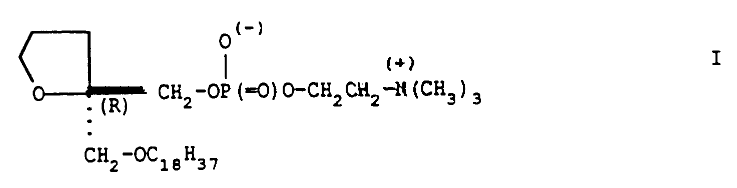 à savoir le 4-oxyde du sel interne de l'hydroxyde de (R)-2-[(2-octadécyloxyméthyltétrahydrofuranne-2-ylméthoxy)hydroxyphosphinyloxy]-N,N,N-triméthyléthanaminium, comprenant l'octadécylation et la phosphorylcholination du monobutyrate de (R)-2,2-bis(hydroxyméthyl)tétrahydrofuranne de formule II 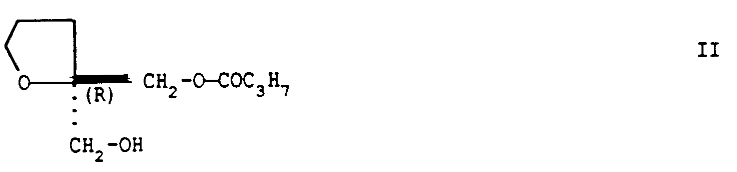 avec les étapes intermédiaires de protection et de déprotection,
selon le schéma réactionnel suivant: 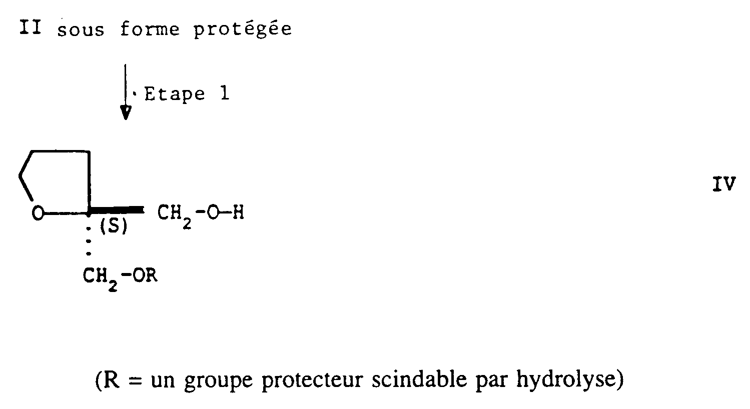 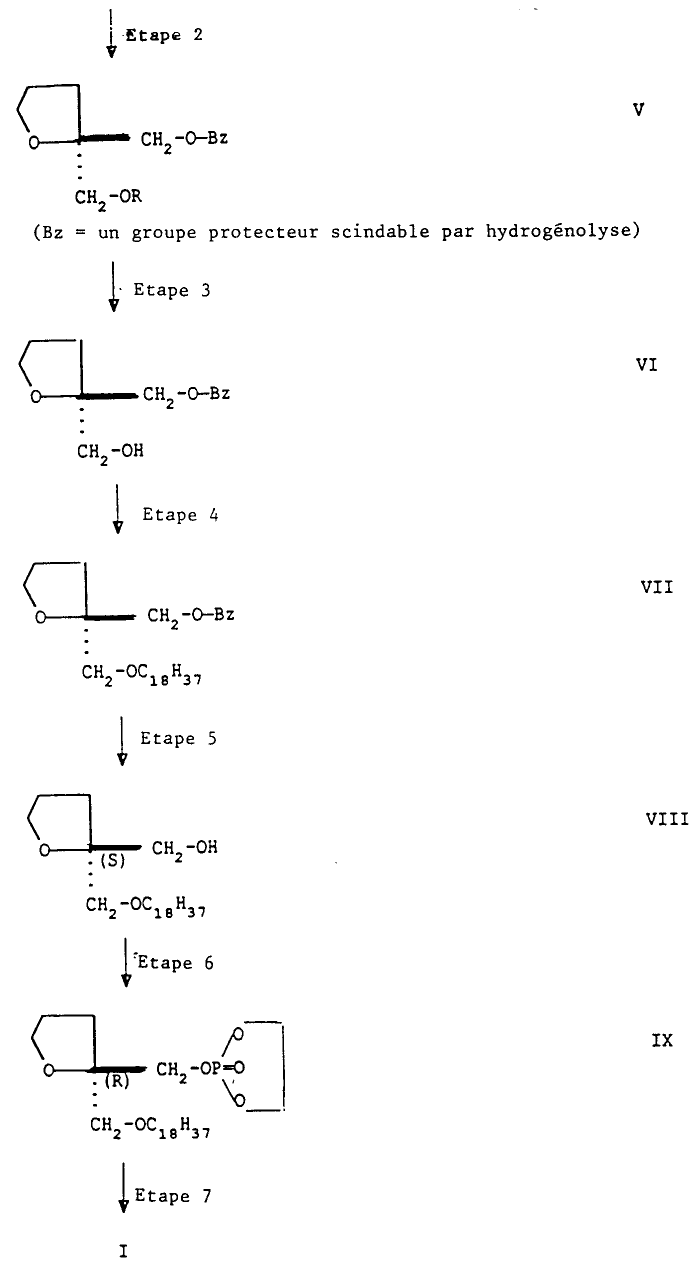 les réactions suivantes étant effectuées:
- Etape 1: hydrolyse alcoolique,
- Etape 2: protection avec un groupe protecteur scindable par hydrogénolyse,
- Etape 3: déprotection hydrolytique,
- Etape 4: alkylation,
- Etape 5: déprotection hydrogénolytique,
- Etape 6: acylation avec le 2-chloro-2-oxo-1,3,2-dioxaphospholane,
- Etape 7: amination avec de la triméthylamine.

2. Un procédé de préparation de l'énantiomère du composé de formule I, selon le schéma réactionnel suivant: 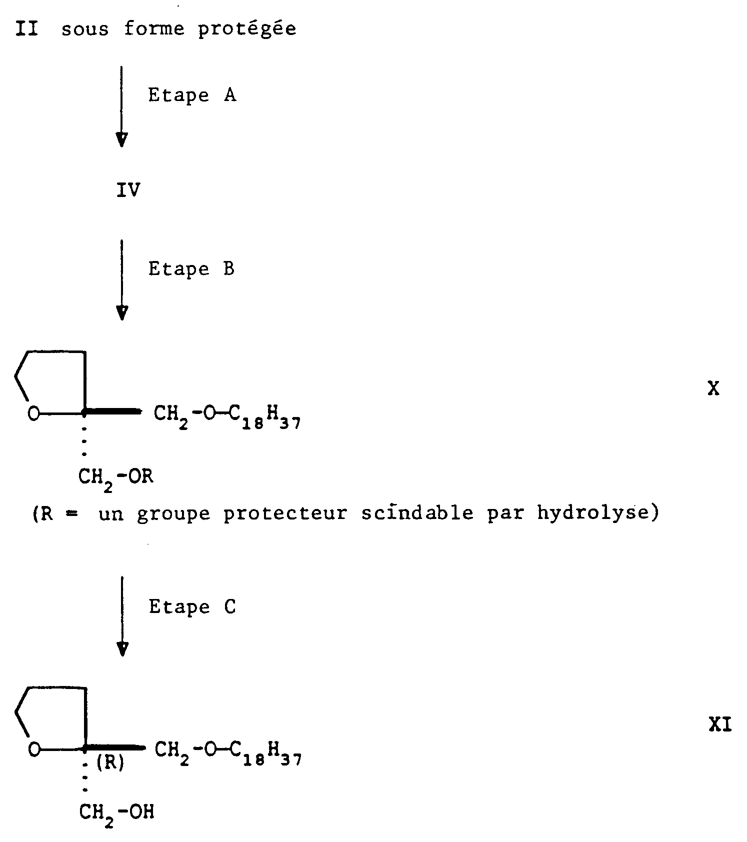 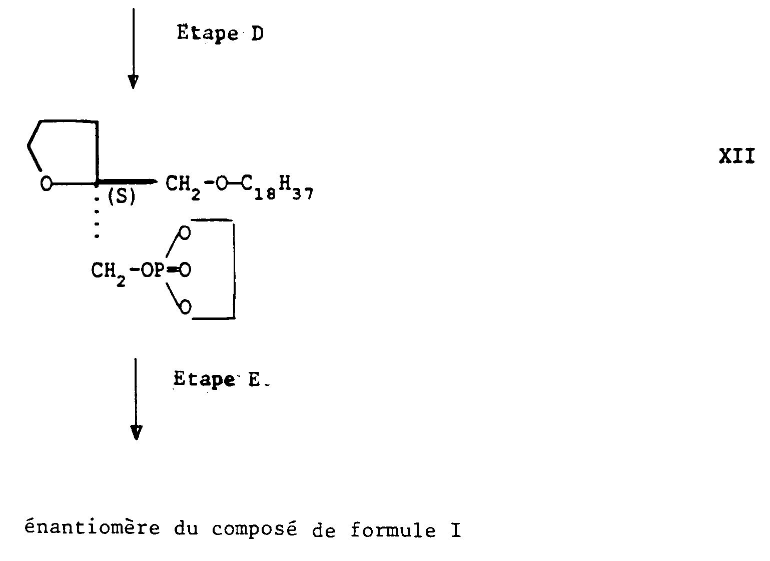 les réactions suivantes étant effectuées:
- Etape A: hydrolyse alcoolique,
- Etape B: alkylation,
- Etape C: déprotection hydrolytique,
- Etape D: acylation avec le 2-chloro-2-oxo-1,3,2,-dioxaphospholane,
- Etape E: amination avec de la triméthylamine.

3. Un procédé selon la revendication 1 ou 2, où le composé de formule II est sous forme protégée et est le composé de formule IIa

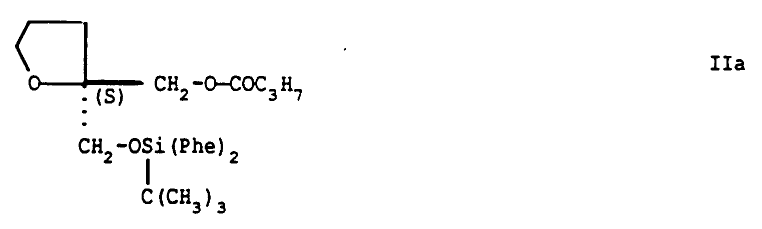

4. Un procédé selon la revendication 1 ou 2 où le composé de formule II est sous forme protégée et est le composé de formule IIb

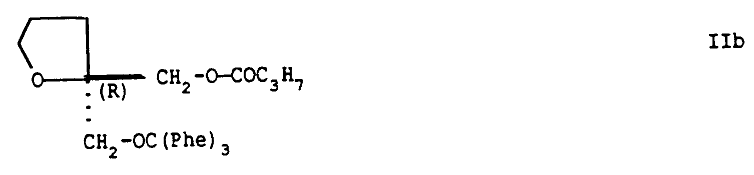

5. Un procédé de préparation du monobutyrate de (R)-2,2-bis(hydroxyméthyl)tétrahydrofuranne de formule II 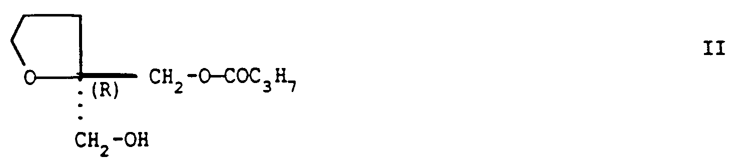 sous forme libre ou sous forme protégée, comprenant la monohydrolyse de manière énantio-spécifique du dibutyrate de 2,2-bis(hydroxyméthyl)tétrahydrofuranne de formule III 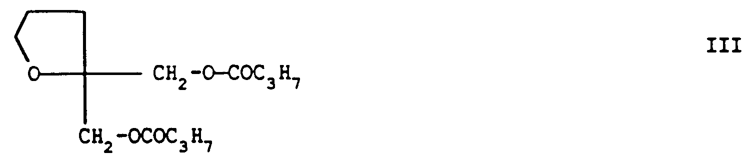 et éventuellement la transformation du composé résultant de formule II sous forme libre en forme protégée.

6. Un procédé selon la revendication 5, comprenant l'hydrolyse enzymatique.

7. Un procédé selon la revendication 5, comprenant l'hydrolyse avec une lipase.

8. Un procédé de préparation d'une composition pharmaceutique, comprenant le mélange d'un composé de formule I tel que défini à la revendication 1 ou son énantiomère et d'au moins un véhicule ou diluant pharmaceutiquement acceptable.

9. Un procédé de préparation d'un composé de formule VIII tel que défini à la revendication 1, comprenant le schéma réactionnel défini à la revendication 1 sans les étapes 6 et 7.

10. Un procédé de préparation d'un composé de formule XI tel que défini à la revendication 2, comprenant le schéma réactionnel défini à la revendication 2 sans les étapes D et E.
